# EUROPEAN PATENT SPECIFICATION

(11) **EP 1 516 928 B1**
(45) Date of publication and mention of the grant of the patent: **17.02.2010**
(21) Application number: 03733573.4
(22) Date of filing: 25.06.2003
(51) Int. Cl.: C12N 15/62, C12N 15/61, C12N 9/90, C12N 1/21, C12P 21/02

(54) **EXPRESSION VECTOR, HOST, FUSED PROTEIN, PROCESS FOR PRODUCING FUSED PROTEIN AND PROCESS FOR PRODUCING PROTEIN**
EXPRESSIONSVEKTOR, WIRT, FUSIONSPROTEIN, VERFAHREN ZUR HERSTELLUNG VON FUSIONSPROTEIN UND VERFAHREN ZUR HERSTELLUNG VON PROTEIN
VECTEUR D'EXPRESSION, HOTE, PROTEINE FUSIONNEE, PROCEDE POUR PRODUIRE UNE PROTEINE FUSIONNEE ET PROCEDE POUR PRODUIRE UNE PROTEINE

(30) Priority: 25.06.2002 JP 2002185020
(43) Date of publication of application: 23.03.2005
(73) Proprietor: SEKISUI CHEMICAL CO., LTD., Osaka-shi, Osaka 530-8565 (JP)
(72) Inventor: IDENO, Akira, c/o Marine Biotechnology Inst. Co., Ltd, Kamaishi-shi, Iwate 026-0001 (JP); MARUYAMA, Tadashi, Marine Biotechnology Inst. Co., Ltd, Kamaishi-shi, Iwate 026-0001 (JP); FURUTANI, Masahiro, c/o Sekisui Chemical CO., Ltd., Mishima-gun, Osaka 618-8589 (JP)
(74) Representative: Jones, Helen M.M.
(86) International application number: PCT/JP2003/008020
(87) International publication number: WO 2004/001041

(56) References cited:
- WO-A-00/20606
- WO-A-97/10253
- WO-A-99/13091
- WO-A-03/000878
- WO-A1-00/75346
- JP-A- 2002 262 883
- JP-A- 2002 306 182
- HOTTENROTT S ET AL: "The Escherichia coli SlyD is a metal ion-regulated peptidyl-prolyl cis/trans-isomerase." THE JOURNAL OF BIOLOGICAL CHEMISTRY. 20 JUN 1997, vol. 272, no. 25, 20 June 1997 (1997-06-20), pages 15697-15701, XP002400328 ISSN: 0021-9258
- IDENO A. ET AL.: 'The 28.3 kDa FK506 binding protein from a thermophilic archaeum, methanobacterium thermoautotrophicum, protects the denaturation of proteins in vitro' EUR. J. BIOCHEM. vol. 267, no. 11, 2000, pages 3139 - 3149, XP002973574
- BEHRENS S. ET AL.: 'The SurA periplasmic PPIase lacking its parvulin domains functions in vivo and has chaperone activity' EMBO J. vol. 20, no. 1-2, 2001, pages 285 - 294, XP002973575
- MARUYAMA T. ET AL.: 'Archaeal peptidyl prolyl cis-trans isomerases (PPIases)' FRONT BIOSCI. vol. 5, 2000, pages D821 - D836, XP002973576
- HUANG G.C. ET AL.: 'Assisted folding of D-glyceraldehyde-3-phosphate dehydrogenase by trigger factor' PROTEIN SCI. vol. 9, no. 6, 2000, pages 1254 - 1261, XP002973577
- ZARNT T. ET AL.: 'Modular structure of the trigger factor required for high activity in protein folding' J. MOL. BIOL. vol. 271, no. 5, 1997, pages 827 - 837, XP004453769
- ARIE J.P. ET AL.: 'Chaperone function of FkpA, a heat shock prolyl isomerase, in the periplasm of escherichia coli' MOL. MICROBIOL. vol. 39, no. 1, 2001, pages 199 - 210, XP002973578
- RATAJCZAK T. ET AL.: 'The cyclophilin component of the unactivated estrogen receptor contains a tetratricopeptide repeat domain and shares identity with p59 (FKBP59)' J. BIOL. CHEM. vol. 268, no. 18, 1993, pages 13187 - 13192, XP002973579
- PIRKL F. ET AL.: 'Functional analysis of the Hsp90-associated human peptidyl prolyl cis/trans isomerases FKBP51, FKBP52 and cyp40' J. MOL. BIOL. vol. 308, no. 4, 2001, pages 795 - 806, XP004480468
- RAMM K. ET AL.: 'The periplasmic escherichia coli peptidylprolylcis, trans-isomerase FkpA. II. Isomerase-independent chaperone activity in vitro' J. BIOL. CHEM. vol. 275, no. 22, 2000, pages 17106 - 17113, XP002973580
- TOSHII IIDA ET AL.: "FK506-binding protein-type peptidyl-prolyl cis-trans isomerase from a halophilic archaeum, Halobacterium cutirubrum" GENE, vol. 256, no. 1-2, 3 October 2000 (2000-10-03), pages 319-326, Elsevier Science B.V., Amsterdam, NL

### SEQUENCE LISTING

<110> Sekisui Chemical Co.,Ltd.
   Marine Biotechnology Co.,Ltd.
<120> EXPRESSION VECTOR, HOST, FUSED PROTEIN, PROCESS, FOR PRODUCING FUSED PROTEIN AND PROCESS FOR PRODUCING PROTEIN
<130> 02P00846
<150> JP P002-185020
   <151> 2002-06-25
<160> 14
<210> 1
   <211> 257
   <212> PRT
   <213> Pyrococcus horikoshii
<400> 1
   Met Lys Val Glu Arg Gly Asp Val Ile Arg Leu His Tyr Thr Gly Arg
   1 5 10 15
   Val Lys Glu Thr Gly Gln Ile Phe Asp Thr Thr Tyr Glu Glu Val Ala
   20 25 30
   Lys Glu Ala Gly Ile Tyr Asn Pro Lys Gly Ile Tyr Gly Pro Val Pro
   35 40 45
   Ile Ile Val Gly Ala Gly His Val Ile Ser Gly Leu Asp Lys Arg Leu
   50 55 60
   Val Gly Leu Glu Val Gly Lys Lys Tyr Thr Leu Glu Val Pro Pro Glu
   65 70 75 80
   Glu Gly Phe Gly Leu Arg Asp Pro Lys Leu Ile Lys Val Phe Thr Met
   85 90 95
   Gly Gln Phe Arg Lys Gln Gly Ile Val Pro Phe Pro Gly Leu Glu Val
   100 105 110
   Glu Val Thr Thr Asp Asn Gly Arg Lys Met Lys Gly Arg Val Ile Thr
   115 120 125
   Val Ser Gly Gly Arg Val Arg Val Asp Phe Asn His Pro Leu Ala Gly
   130 135 140
   Lys Thr Leu Ile Tyr Glu Val Glu Ile Val Glu Lys Ile Glu Asp Pro
   145 150 155 160
   Ile Glu Lys Ile Lys Ala Leu Ile Glu Leu Arg Leu Pro Met Ile Asp
   165 170 175
   Arg Asp Lys Val Ile Ile Glu Val Gly Glu Lys Asp Val Lys Val Asn
   180 185 190
   Phe Gly Glu Gln Asp Val Asp Pro Lys Thr Leu Ile Leu Gly Glu Ile
   195 200 205
   Leu Leu Glu Ser Asp Ile Lys Phe Leu Gly Tyr Glu Lys Val Glu Phe
   210 215 220
   Lys Pro Ser Val Glu Glu Leu Leu Arg Pro Lys Gln Glu Glu Pro Val
   225 230 235 240
   Glu Glu Glu Lys Lys Glu Glu Gln Glu Glu Ser Glu Glu Ala Gln Ser
   245 250 255
   Ser
<210> 2
   <211> 157
   <212> PRT
   <213> Methanococcus jannaschii
<400> 2
   Leu Ile Asn Leu Ile Lys Lys Gly Asp Tyr Val Lys Val Asp Tyr Ile
   1 5 10 15
   Leu Glu Val Asp Gly Lys Val Ile Asp Thr Ser Ile Glu Glu Val Ala
   20 25 30
   Lys Glu Asn Lys Ile Tyr Tyr Pro Glu Arg Glu Tyr Glu Pro Ile Gly
   35 40 45
   Phe Ile Val Gly Asn Gly Glu Leu Ile Glu Gly Phe Glu Glu Ala Val
   50 55 60
   Ile Gly Met Glu Val Gly Glu Glu Lys Thr Val Thr Ile Pro Pro Glu
   65 70 75 80
   Lys Gly Tyr Gly Leu Arg Asp Glu Arg Leu Ile Gln Glu Ile Pro Lys
   85 90 95
   Glu Met Phe Ala Asp Ala Asp Phe Glu Pro Gln Glu Gly Met Leu Ile
   100 105 110
   Leu Ala Ser Gly Ile Pro Ala Lys Ile Ile Lys Val Thr Asp Asp Thr
   115 120 125
   Val Thr Leu Asp Phe Asn His Glu Leu Ala Gly Lys Glu Leu Lys Phe
   130 135 140
   Thr Ile Lys Val Arg Asp Val Gln Pro Ala Glu Ser Glu
   145 150 155
<210> 3
   <211> 432
   <212> PRT
   <213> Escerichia coli
<400> 3
   Met Gln Val Ser Val Glu Thr Thr Gln Gly Leu Gly Arg Arg Val Thr
   1 5 10 15
   Ile Thr Ile Ala Ala Asp Ser Ile Glu Thr Ala Val Lys Ser Glu Leu
   20 25 30
   Val Asn Val Ala Lys Lys Val Arg Ile Asp Gly Phe Arg Lys Gly Lys
   35 40 45
   Val Pro Met Asn Ile Val Ala Gln Arg Tyr Gly Ala Ser Val Arg Gln
   50 55 60
   Asp Val Leu Gly Asp Leu Met Ser Arg Asn Phe Ile Asp Ala Ile Ile
   65 70 75 80
   Lys Glu Lys Ile Asn Pro Ala Gly Ala Pro Thr Tyr Val Pro Gly Glu
   85 90 95
   Tyr Lys Leu Gly Glu Asp Phe Thr Tyr Ser Val Glu Phe Glu Val Tyr
   100 105 110
   Pro Glu Val Glu Leu Gln Gly Leu Glu Ala Ile Glu Val Glu Lys Pro
   115 120 125
   Ile Val Glu Val Thr Asp Ala Asp Val Asp Gly Met Leu Asp Thr Leu
   130 135 140
   Arg Lys Gln Gln Ala Thr Trp Lys Glu Lys Asp Gly Ala Val Glu Ala
   145 150 155 160
   Glu Asp Arg Val Thr Ile Asp Phe Thr Gly Ser Val Asp Gly Glu Glu
   165 170 175
   Phe Glu Gly Gly Lys Ala Ser Asp Phe Val Leu Ala Met Gly Gln Gly
   180 185 190
   Arg Met Ile Pro Gly Phe Glu Asp Gly Ile Lys Gly His Lys Ala Gly
   195 200 205
   Glu Glu Phe Thr Ile Asp Val Thr Phe Pro Glu Glu Tyr His Ala Glu
   210 215 220
   Asn Leu Lys Gly Lys Ala Ala Lys Phe Ala Ile Asn Leu Lys Lys Val
   225 230 235 240
   Glu Glu Arg Glu Leu Pro Glu Leu Thr Ala Glu Phe Ile Lys Arg Phe
   245 250 255
   Gly Val Glu Asp Gly Ser Val Glu Gly Leu Arg Ala Glu Val Arg Lys
   260 265 270
   Asn Met Glu Arg Glu Leu Lys Ser Ala Ile Arg Asn Arg Val Lys Ser
   275 280 285
   Gln Ala Ile Glu Gly Leu Val Lys Ala Asn Asp Ile Asp Val Pro Ala
   290 295 300
   Ala Leu Ile Asp Ser Glu Ile Asp Val Leu Arg Arg Gln Ala Ala Gln
   305 310 315 320
   Arg Phe Gly Gly Asn Glu Lys Gln Ala Leu Glu Leu Pro Arg Glu Leu
   325 330 335
   Phe Glu Glu Gln Ala Lys Arg Arg Val Val Val Gly Leu Leu Leu Gly
   340 345 350
   Glu Val Ile Arg Thr Asn Glu Leu Lys Ala Asp Glu Glu Arg Val Lys
   355 360 365
   Gly Leu Ile Glu Glu Met Ala Ser Ala Tyr Glu Asp Pro Lys Glu Val
   370 375 380
   Ile Glu Phe Tyr Ser Lys Asn Lys Glu Leu Met Asp Asn Met Arg Asn
   385 390 395 400
   Val Ala Leu Glu Glu Gln Ala Val Glu Ala Val Leu Ala Lys Ala Lys
   405 410 415
   Val Thr Glu Lys Glu Thr Thr Phe Asn Glu Leu Met Asn Gln Gln Ala
   420 425 430
<210> 4
   <211> 1299
   <212> DNA
   <213> Escerichia coli
<400> 4
<210> 5
   <211> 270
   <212> PRT
   <213> Escerichia coli
<400> 5
   Met Lys Ser Leu Phe Lys Val Thr Leu Leu Ala Thr Thr Met Ala Val
   1 5 10 15
   Ala Leu His Ala Pro Ile Thr Phe Ala Ala Glu Ala Ala Lys Pro Ala
   20 25 30
   Thr Ala Ala Asp Ser Lys Ala Ala Phe Lys Asn Asp Asp Gln Lys Ser
   35 40 45
   Ala Tyr Ala Leu Gly Ala Ser Leu Gly Arg Tyr Met Glu Asn Ser Leu
   50 55 60
   Lys Glu Gln Glu Lys Leu Gly Ile Lys Leu Asp Lys Asp Gln Leu Ile
   65 70 75 80
   Ala Gly Val Gln Asp Ala Phe Ala Asp Lys Ser Lys Leu Ser Asp Gln
   85 90 95
   Glu Ile Glu Gln Thr Leu Gln Ala Phe Glu Ala Arg Val Lys Ser Ser
   100 105 110
   Ala Gln Ala Lys Met Glu Lys Asp Ala Ala Asp Asn Glu Ala Lys Gly
   115 120 125
   Lys Glu Tyr Arg Glu Lys Phe Ala Lys Glu Lys Gly Val Lys Thr Ser
   130 135 140
   Ser Thr Gly Leu Val Tyr Gln Val Val Glu Ala Gly Lys Gly Glu Ala
   145 150 155 160
   Pro Lys Asp Ser Asp Thr Val Val Val Asn Tyr Lys Gly Thr Leu Ile
   165 170 175
   Asp Gly Lys Glu Phe Asp Asn Ser Tyr Thr Arg Gly Glu Pro Leu Ser
   180 185 190
   Phe Arg Leu Asp Gly Val Ile Pro Gly Trp Thr Glu Gly Leu Lys Asn
   195 200 205
   Ile Lys Lys Gly Gly Lys Ile Lys Leu Val Ile Pro Pro Glu Leu Ala
   210 215 220
   Tyr Gly Lys Ala Gly Val Pro Gly Ile Pro Pro Asn Ser Thr Leu Val
   225 230 235 240
   Phe Asp Val Glu Leu Leu Asp Val Lys Pro Ala Pro Lys Ala Asp Ala
   245 250 255
   Lys Pro Glu Ala Asp Ala Lys Ala Ala Asp Ser Ala Lys Lys
   260 265 270
<210> 6
   813
   <212> DNA
   <213> Escerichia coli
<400> 6
<210> 7
   <211> 428
   <212> PRT
   <213> Escerichia coli
<400> 7
   Met Lys Asn Trp Lys Thr Leu Leu Leu Gly Ile Ala Met Ile Ala Asn
   1 5 10 15
   Thr Ser Phe Ala Ala Pro Gln Val Val Asp Lys Val Ala Ala Val Val
   20 25 30
   Asn Asn Gly Val Val Leu Glu Ser Asp Val Asp Gly Leu Met Gln Ser
   35 40 45
   Val Lys Leu Asn Ala Ala Gln Ala Arg Gln Gln Leu Pro Asp Asp Ala
   50 55 60
   Thr Leu Arg His Gln Ile Met Glu Arg Leu Ile Met Asp Gln Ile Ile
   65 70 75 80
   Leu Gln Met Gly Gln Lys Met Gly Val Lys Ile Ser Asp Glu Gln Leu
   85 90 95
   Asp Gln Ala Ile Ala Asn Ile Ala Lys Gln Asn Asn Met Thr Leu Asp
   100 105 110
   Gln Met Arg Ser Arg Leu Ala Tyr Asp Gly Leu Asn Tyr Asn Thr Tyr
   115 120 125
   Arg Asn Gln Ile Arg Lys Glu Met Ile Ile Ser Glu Val Arg Asn Asn
   130 135 140
   Glu Val Arg Arg Arg Ile Thr Ile Leu Pro Gln Glu Val Glu Ser Leu
   145 150 155 160
   Ala Gln Gln Val Gly Asn Gln Asn Asp Ala Ser Thr Glu Leu Asn Leu
   165 170 175
   Ser His Ile Leu Ile Pro Leu Pro Glu Asn Pro Thr Ser Asp Gln Val
   180 185 190
   Asn Glu Ala Glu Ser Gln Ala Arg Ala Ile Val Asp Gln Ala Arg Asn
   195 200 205
   Gly Ala Asp Phe Gly Lys Leu Ala Ile Ala His Ser Ala Asp Gln Gln
   210 215 220
   Ala Leu Asn Gly Gly Gln Met Gly Trp Gly Arg Ile Gln Glu Leu Pro
   225 230 235 240
   Gly Ile Phe Ala Gln Ala Leu Ser Thr Ala Lys Lys Gly Asp Ile Val
   245 250 255
   Gly Pro Ile Arg Ser Gly Val Gly Phe His Ile Leu Lys Val Asn Asp
   260 265 270
   Leu Arg Gly Glu Ser Lys Asn Ile Ser Val Thr Glu Val His Ala Arg
   275 280 285
   His Ile Leu Leu Lys Pro Ser Pro Ile Met Thr Asp Glu Gln Ala Arg
   290 295 300
   Val Lys Leu Glu Gln Ile Ala Ala Asp Ile Lys Ser Gly Lys Thr Thr
   305 310 315 320
   Phe Ala Ala Ala Ala Lys Glu Phe Ser Gln Asp Pro Gly Ser Ala Asn
   325 330 335
   Gln Gly Gly Asp Leu Gly Trp Ala Thr Pro Asp Ile Phe Asp Pro Ala
   340 345 350
   Phe Arg Asp Ala Leu Thr Arg Leu Asn Lys Gly Gln Met Ser Ala Pro
   355 360 365
   Val His Ser Ser Phe Gly Trp His Leu Ile Glu Leu Leu Asp Thr Arg
   370 375 380
   Asn Val Asp Lys Thr Asp Ala Ala Gln Lys Asp Arg Ala Tyr Arg Met
   385 390 395 400
   Leu Met Asn Arg Lys Phe Ser Glu Glu Ala Ala Ser Trp Met Gln Glu
   405 410 415
   Gln Arg Ala Ser Ala Tyr Val Lys Ile Leu Ser Asn
   420 425
<210> 8
   <211> 1287
   <212> DNA
   <213> Escerichia coli
<400> 8
   atgaagaact ggaaaacgct gcttctcggt atcgccatga tcgcgaatac cagtttcgct 60
   gccccccagg tagtcgataa agtcgcagcc gtcgtcaata acggcgtcgt gctggaaagc 120
   gacgttgatg gattaatgca gtcggtaaaa ctgaacgctg ctcaggcaag gcagcaactt 180
   cctgatgacg cgacgctgcg ccaccaaatc atggaacgtt tgatcatgga tcaaatcatc 240
   ctgcagatgg ggcagaaaat gggagtgaaa atctccgatg agcagctgga tcaggcgatt 300
   gctaacatcg cgaaacagaa caacatgacg ctggatcaga tgcgcagccg tctggcttac 360
   gatggactga actacaacac ctatcgtaac cagatccgca aagagatgat tatctctgaa 420
   gtgcgtaaca acgaggtgcg tcgtcgcatc accatcctgc cgcaggaagt cgaatccctg 480
   gcgcagcagg tgggtaacca aaacgacgcc agcactgagc tgaacctgag ccacatcctg 540
   atcccgctgc cggaaaaccc gacctctgat caggtgaacg aagcggaaag ccaggcgcgc 600
   gccattgtcg atcaggcgcg taacggcgct gatttcggta agctggcgat tgctcattct 660
   gccgaccagc aggcgctgaa cggcggccag atgggctggg gccgtattca ggagttgccc 720
   gggatcttcg cccaggcatt aagcaccgcg aagaaaggcg acattgttgg cccgattcgt 780
   tccggcgttg gcttccatat tctgaaagtt aacgacctgc gcggcgaaag caaaaatatc 840
   tcggtgaccg aagttcatgc tcgccatatt ctgctgaaac cgtcgccgat catgactgac 900
   gaacaggccc gtgtgaaact ggaacagatt gctgctgata tcaagagtgg taaaacgact 960
   tttgctgccg cagcgaaaga gttctctcag gatccaggct ctgctaacca gggcggcgat 1020
   ctcggctggg ctacaccaga tattttcgat ccggccttcc gtgacgccct gactcgcctg 1080
   aacaaaggtc aaatgagtgc accggttcac tcttcattcg gctggcattt aatcgaactg 1140
   ctggataccc gtaatgtcga taaaaccgac gctgcgcaga aagatcgtgc ataccgcatg 1200
   ctgatgaacc gtaagttctc ggaagaagca gcaagctgga tgcaggaaca acgtgccagc 1260
   gcctacgtta aaatcctgag caactaa 1287
<210> 9
   <211> 459
   <212> PRT
   <213> human
<400> 9
   Met Thr Ala Glu Glu Met Lys Ala Thr Glu Ser Gly Ala Gln Ser Ala
   1 5 10 15
   Pro Leu Pro Met Glu Gly Val Asp Ile Ser Pro Lys Gln Asp Glu Gly
   20 25 30
   Val Leu Lys Val Ile Lys Arg Glu Gly Thr Gly Thr Glu Met Pro Met
   35 40 45
   Ile Gly Asp Arg Val Phe Val His Tyr Thr Gly Trp Leu Leu Asp Gly
   50 55 60
   Thr Lys Phe Asp Ser Ser Leu Asp Arg Lys Asp Lys Phe Ser Phe Asp
   65 70 75 80
   Leu Gly Lys Gly Glu Val Ile Lys Ala Trp Asp Ile Ala Ile Ala Thr
   85 90 95
   Met Lys Val Gly Glu Val Cys His Ile Thr Cys Lys Pro Glu Tyr Ala
   100 105 110
   Tyr Gly Ser Ala Gly Ser Pro Pro Lys Ile Pro Pro Asn Ala Thr Leu
   115 120 125
   Val Phe Glu Val Glu Leu Phe Glu Phe Lys Gly Glu Asp Leu Thr Glu
   130 135 140
   Glu Glu Asp Gly Gly Ile Ile Arg Arg Ile Gln Thr Arg Gly Glu Gly
   145 150 155 160
   Tyr Ala Lys Pro Asn Glu Gly Ala Ile Val Glu Val Ala Leu Glu Gly
   165 170 175
   Tyr Tyr Lys Asp Lys Leu Phe Asp Gln Arg Glu Leu Arg Phe Glu Ile
   180 185 190
   Gly Glu Gly Glu Asn Leu Asp Leu Pro Tyr Gly Leu Glu Arg Ala Ile
   195 200 205
   Gln Arg Met Glu Lys Gly Glu His Ser Ile Val Tyr Leu Lys Pro Ser
   210 215 220
   Tyr Ala Phe Gly Ser Val Gly Lys Glu Lys Phe Gln Ile Pro Pro Asn
   225 230 235 240
   Ala Glu Leu Lys Tyr Glu Leu His Leu Lys Ser Phe Glu Lys Ala Lys
   245 250 255
   Glu Ser Trp Glu Met Asn Ser Glu Glu Lys Leu Glu Gln Ser Thr Ile
   260 265 270
   Val Lys Glu Arg Gly Thr Val Tyr Phe Lys Glu Gly Lys Tyr Lys Gln
   275 280 285
   Ala Leu Leu Gln Tyr Lys Lys Ile Val Ser Trp Leu Glu Tyr Glu Ser
   290 295 300
   Ser Phe Ser Asn Glu Glu Ala Gln Lys Ala Gln Ala Leu Arg Leu Ala
   305 310 315 320
   Ser His Leu Asn Leu Ala Met Cys His Leu Lys Leu Gln Ala Phe Ser
   325 330 335
   Ala Ala Ile Glu Ser Cys Asn Lys Ala Leu Glu Leu Asp Ser Asn Asn
   340 345 350
   Glu Lys Gly Leu Phe Arg Arg Gly Glu Ala His Leu Ala Val Asn Asp
   355 360 365
   Phe Glu Leu Ala Arg Ala Asp Phe Gln Lys Val Leu Gln Leu Tyr Pro
   370 375 380
   Asn Asn Lys Ala Ala Lys Thr Gln Leu Ala Val Cys Gln Gln Arg Ile
   385 390 395 400
   Arg Arg Gln Leu Ala Arg Glu Lys Lys Leu Tyr Ala Asn Met Phe Glu
   405 410 415
   Arg Leu Ala Glu Glu Glu Asn Lys Ala Lys Ala Glu Ala Ser Ser Gly
   420 425 430
   Asp His Pro Thr Asp Thr Glu Met Lys Glu Glu Gln Lys Ser Asn Thr
   435 440 445
   Ala Gly Ser Gln Ser Gln Val Glu Thr Glu Ala
   450 455
<210> 10
   <211> 1380
   <212> DNA
   <213> human
<400> 10
   atgacagccg aggagatgaa ggcgaccgag agcggggcgc agtcggcgcc gctgcccatg 60
   gagggagtgg acatcagccc caaacaggac gaaggcgtgc tgaaggtcat caagagagag 120
   ggcacaggta cagagatgcc catgattggg gaccgagtct ttgtccacta cactggctgg 180
   ctattagatg gcacaaagtt tgactccagt ctggatcgca aggacaaatt ctcctttgac 240
   ctgggaaaag gggaggtcat caaggcttgg gacattgcca tagccaccat gaaggtgggg 300
   gaggtgtgcc acatcacctg caaaccagaa tatgcctacg gttcagcagg cagtcctcca 360
   aagattcccc ccaatgccac gcttgtattt gaggtggagt tgtttgagtt taagggagaa 420
   gatctgacgg aagaggaaga tggcggaatc attcgcagaa tacagactcg cggtgaaggc 480
   tatgctaagc ccaatgaggg tgctatcgtg gaggttgcac tggaagggta ctacaaggac 540
   aagctctttg accagcggga gctccgcttt gagattggcg agggggagaa cctggatctg 600
   ccttatggtc tggagagggc cattcagcgc atggagaaag gagaacattc catcgtgtac 660
   ctcaagccca gctatgcttt tggcagtgtt gggaaggaaa agttccaaat cccaccaaat 720
   gctgagctga aatatgaatt acacctcaag agttttgaaa aggccaagga gtcttgggag 780
   atgaattcag aagagaagct ggaacagagc accatagtga aagagcgggg cactgtgtac 840
   ttcaaggaag gtaaatacaa gcaagcttta ctacagtata agaagatcgt gtcttggctg 900
   gaatatgagt ctagtttttc caatgaggaa gcacagaaag cacaggccct tcgactggcc 960
   tctcacctca acctggccat gtgtcatctg aaactacagg ccttctctgc tgccattgaa 1020
   agctgtaaca aggccctaga actggacagc aacaacgaga agggcctctt ccgccgggga 1080
   gaggcccacc tggccgtgaa tgactttgaa ctggcacggg ctgatttcca gaaggtcctg 1140
   cagctctacc ccaacaacaa agccgccaag acccagctgg ctgtgtgcca gcagcggatc 1200
   cgaaggcagc ttgcccggga gaagaagctc tatgccaata tgtttgagag gctggctgag 1260
   gaggagaaca aggccaaggc agaggcttcc tcaggagacc atcccactga cacagagatg 1320
   aaggaggagc agaagagcaa cacggcaggg agccagtctc aggtggagac agaagcatag 1380
<210> 11
   <211> 370
   <212> PRT
   <213> human
<400> 11
   Met Ser His Pro Ser Pro Gln Ala Lys Pro Ser Asn Pro Ser Asn Pro
   1 5 10 15
   Arg Val Phe Phe Asp Val Asp Ile Gly Gly Glu Arg Val Gly Arg Ile
   20 25 30
   Val Leu Glu Leu Phe Ala Asp Ile Val Pro Lys Thr Ala Glu Asn Phe
   35 40 45
   Arg Ala Leu Cys Thr Gly Glu Lys Gly Ile Gly His Thr Thr Gly Lys
   50 55 60
   Pro Leu His Phe Lys Gly Cys Pro Phe His Arg Ile Ile Lys Lys Phe
   65 70 75 80
   Met Ile Gln Gly Gly Asp Phe Ser Asn Gln Asn Gly Thr Gly Gly Glu
   85 90 95
   Ser Ile Tyr Gly Glu Lys Phe Glu Asp Glu Asn Phe His Tyr Lys His
   100 105 110
   Asp Arg Glu Gly Leu Leu Ser Met Ala Asn Ala Gly Arg Asn Thr Asn
   115 120 125
   Gly Ser Gln Phe Phe Ile Thr Thr Val Pro Thr Pro His Leu Asp Gly
   130 135 140
   Lys His Val Val Phe Gly Gln Val Ile Lys Gly Ile Gly Val Ala Arg
   145 150 155 160
   Ile Leu Glu Asn Val Glu Val Lys Gly Glu Lys Pro Ala Lys Leu Cys
   165 170 175
   Val Ile Ala Glu Cys Gly Glu Leu Lys Glu Gly Asp Asp Gly Gly Ile
   180 185 190
   Phe Pro Lys Asp Gly Ser Gly Asp Ser His Pro Asp Phe Pro Glu Asp
   195 200 205
   Ala Asp Ile Asp Leu Lys Asp Val Asp Lys Ile Leu Leu Ile Thr Glu
   210 215 220
   Asp Leu Lys Asn Ile Gly Asn Thr Phe Phe Lys Ser Gln Asn Trp Glu
   225 230 235 240
   Met Ala Ile Lys Lys Tyr Ala Glu Val Leu Arg Tyr Val Asp Ser Ser
   245 250 255
   Lys Ala Val Ile Glu Thr Ala Asp Arg Ala Lys Leu Gln Pro Ile Ala
   260 265 270
   Leu Ser Cys Val Leu Asn Ile Gly Ala Cys Lys Leu Lys Met Ser Asn
   275 280 285
   Trp Gln Gly Ala Ile Asp Ser Cys Leu Glu Ala Leu Glu Leu Asp Pro
   290 295 300
   Ser Asn Thr Lys Ala Leu Tyr Arg Arg Ala Gln Gly Trp Gln Gly Leu
   305 310 315 320
   Lys Glu Tyr Asp Gln Ala Leu Ala Asp Leu Lys Lys Ala Gln Gly Ile
   325 330 335
   Ala Pro Glu Asp Lys Ala Ile Gln Ala Glu Leu Leu Lys Val Lys Gln
   340 345 350
   Lys Ile Lys Ala Gln Lys Asp Lys Glu Lys Ala Val Tyr Ala Lys Met
   355 360 365
   Phe Ala
   370
<210> 12
   <211> 1113
   <212> DNA
   <213> human
<400> 12
<210> 13
   <211> 422
   <212> PRT
   <213> human
<400> 13
   Met Asp Val Leu Ser Pro Gly Gln Gly Asn Asn Thr Thr Ser Pro Pro
   5 10 15
   Ala Pro Phe Glu Thr Gly Gly Asn Thr Thr Gly Ile Ser Asp Val Thr
   20 25 30
   Val Ser Tyr Gln Val Ile Thr Ser Leu Leu Leu Gly Thr Leu Ile Phe
   35 40 45
   Cys Ala Val Leu Gly Asn Ala Cys Val Val Ala Ala Ile Ala Leu Glu
   50 55 60
   Arg Ser Leu Gln Asn Val Ala Asn Tyr Leu Ile Gly Ser Leu Ala Val
   65 70 75 80
   Thr Asp Leu Met Val Ser Val Leu Val Leu Pro Met Ala Ala Leu Tyr
   85 90 95
   Gln Val Leu Asn Lys Trp Thr Leu Gly Gln Val Thr Cys Asp Leu Phe
   100 105 110
   Ile Ala Leu Asp Val Leu Cys Cys Thr Ser Ser Ile Leu His Leu Cys
   115 120 125
   Ala Ile Ala Leu Asp Arg Tyr Trp Ala Ile Thr Asp Pro Ile Asp Tyr
   130 135 140
   Val Asn Lys Arg Thr Pro Arg Arg Ala Ala Ala Leu Ile Ser Leu Thr
   145 150 155 160
   Trp Leu Ile Gly Phe Leu Ile Ser Ile Pro Pro Met Leu Gly Trp Arg
   165 170 175
   Thr Pro Glu Asp Arg Ser Asp Pro Asp Ala Cys Thr Ile Ser Lys Asp
   180 185 190
   His Gly Tyr Thr Ile Tyr Ser Thr Phe Gly Ala Phe Tyr Ile Pro Leu
   195 200 205
   Leu Leu Met Leu Val Leu Tyr Gly Arg Ile Phe Arg Ala Ala Arg Phe
   210 215 220
   Arg Ile Arg Lys Thr Val Lys Lys Val Glu Lys Thr Gly Ala Asp Thr
   225 230 235 240
   Arg His Gly Ala Ser Pro Ala Pro Gln Pro Lys Lys Ser Val Asn Gly
   245 250 255
   Glu Ser Gly Ser Arg Asn Trp Arg Leu Gly Val Glu Ser Lys Ala Gly
   260 265 270
   Gly Ala Leu Cys Ala Asn Gly Ala Val Arg Gln Gly Asp Asp Gly Ala
   275 280 285
   Ala Leu Glu Val Ile Glu Val His Arg Val Gly Asn Ser Lys Glu His
   290 295 300
   Leu Pro Leu Pro Ser Glu Ala Gly Pro Thr Pro Cys Ala Pro Ala Ser
   305 310 315 320
   Phe Glu Arg Lys Asn Glu Arg Asn Ala Glu Ala Lys Arg Lys Met Ala
   325 330 335
   Leu Ala Arg Glu Arg Lys Thr Val Lys Thr Leu Gly Ile Ile Met Gly
   340 345 350
   Thr Phe Ile Leu Cys Trp Leu Pro Phe Phe Ile Val Ala Leu Val Leu
   355 360 365
   Pro Phe Cys Glu Ser Ser Cys His Met Pro Thr Leu Leu Gly Ala Ile
   370 375 380
   Ile Asn Trp Leu Gly Tyr Ser Asn Ser Leu Leu Asn Pro Val Ile Tyr
   385 390 395 400
   Ala Tyr Phe Asn Lys Asp Phe Gln Asn Ala Phe Lys Lys Ile Ile Lys
   405 410 415
   Cys Lys Phe Cys Arg Gln
   420
<210> 14
   <211> 1266
   <212> DNA
   <213> human
<400> 14
   atggatgtgc tcagccctgg tcagggcaac aacaccacat caccaccggc tccctttgag 60
   accggcggca acactactgg tatctccgac gtgaccgtca gctaccaagt gatcacctct 120
   ctgctgctgg gcacgctcat cttctgcgcg gtgctgggca atgcgtgcgt ggtggctgcc 180
   atcgccttgg agcgctccct gcagaacgtg gccaattatc ttattggctc tttggcggtc 240
   accgacctca tggtgtcggt gttggtgctg cccatggccg cgctgtatca ggtgctcaac 300
   aagtggacac tgggccaggt aacctgcgac ctgttcatcg ccctcgacgt gctgtgctgc 360
   acctcatcca tcttgcacct gtgcgccatc gcgctggaca ggtactgggc catcacggac 420
   cccatcgact acgtgaacaa gaggacgccc cggcgcgccg ctgcgctcat ctcgctcact 480
   tggcttattg gcttcctcat ctctatcccg cccatgctgg gctggcgcac cccggaagac 540
   cgctcggacc ccgacgcatg caccattagc aaggatcatg gctacactat ctattccacc 600
   tttggagctt tctacatccc gctgctgctc atgctggttc tctatgggcg catattccga 660
   gctgcgcgct tccgcatccg caagacggtc aaaaaggtgg agaagaccgg agcggacacc 720
   cgccatggag catctcccgc cccgcagccc aagaagagtg tgaatggaga gtcggggagc 780
   aggaactgga ggctgggcgt ggagagcaag gctgggggtg ctctgtgcgc caatggcgcg 840
   gtgaggcaag gtgacgatgg cgccgccctg gaggtgatcg aggtgcaccg agtgggcaac 900
   tccaaagagc acttgcctct gcccagcgag gctggtccta ccccttgtgc ccccgcctct 960
   ttcgagagga aaaatgagcg caacgccgag gcgaagcgca agatggccct ggcccgagag 1020
   aggaagacag tgaagacgct gggcatcatc atgggcacct tcatcctctg ctggctgccc 1080
   ttcttcatcg tggctcttgt tctgcccttc tgcgagagca gctgccacat gcccaccctg 1140
   ttgggcgcca taatcaattg gctgggctac tccaactctc tgcttaaccc cgtcatttac 1200
   gcatacttca acaaggactt tcaaaacgcg tttaagaaga tcattaagtg taagttctgc 1260
   cgccag 1266

## Description

### TECHNICAL FIELD

The present invention relates to an expression vector, a host, a fused protein, a protein, a process for producing a fused protein and a process for producing a protein, which can prevent expression of a recombinant protein as an abnormal type of an inclusion body and the like, and can produce a recombinant protein as a natural type in a soluble fraction.

### BACKGROUND ART

Recently, genome analysis of various organisms has been completed, and it is considered that, from now on, study progresses towards covering functional analysis of a protein which is an expression product of a gene. Study in assisting analysis of life phenomenon by revealing property of individual proteins and, at the same time, analyzing interaction between proteins comprehensively has been rapidly increased. On the other hand, an intracellular receptor protein which specifically binds to various physiological active substances and transmits its action attracts an important interest in determining its three-dimensional structure because an active substance which binds to the receptor protein can be a candidate substance of a new medicament, and this protein is given an attention in screening of a novel medicament. When one tries to determine property of such the protein, such a method is general that a corresponding gene is inserted on a vector gene, transformed into a host such as bacterium, yeast and insect cell, and property of a recombinant protein obtained by expression is investigated.

Upon assessment of correct property of a protein, either the protein is folded into a correct steric structure or not becomes very important. However, when one tries to make a protein derived from a heterogenous organism by a protein expression method using the aforementioned host expression system, one often encounters the case where only an abnormal type protein having a different steric structure is obtained due to abnormal folding of a protein. It is known that such the protein is expressed as an aggregate called inclusion body in a host, and is degraded with a protease of a host cell. In order to solve them, it is considered that it is extremely important to control so that a reaction of folding a desired protein in a host cell is correctly performed.

When a desired protein is expressed as an inclusion body which is an abnormal type protein, as means for obtaining its normal type, a method of converting it into a normal type in vitro has been previously general. That is, this is a method of recovering an inclusion body from a host, solubilizing the inclusion body with guanidine hydrochloride or urea at a high concentration, and diluting with a suitable buffer to around 30 to 100-fold to re-fold a solubilized desired protein. As one example, an antibody is expected to be utilized in the medical field, but it is known that, when one tries to express its recombinant in a cytoplasm using Escherichia coli as a host, almost all of the recombinant is expressed as an insoluble inclusion body (Pluckthun, Biotechnology, 9, 545-, 1991). As a method of effectively re-folding an antibody obtained as an inclusion body in vitro, there is proposed a method of increasing a yield of a protein by containing chaperonin which promotes re-folding of the protein in a diluent (Japanese Kokai Publication Hei-9-220092). In addition to an antibody, various devices for increasing a yield of a desired protein by adding a reducing agent or an organic acid to a diluent have been proposed, such as a method of re-folding a NGF/BDNF family protein which is expressed as an inclusion body protein (Japanese Kokai Publication Hei-6-327489, Japanese Kokai Publication Hei-6-319549), and a method of re-folding neurotrophin-3 (Japanese Kokai Publication Hei-9-262093). However, these methods of re-folding a protein obtained as an inclusion body in vitro take a long time, but a resulting yield is low.

WO99/13091 discloses fusion genes for expression heterologous proteins comprising a gene encoding a heterologous protein and a gene encoding an E.coli carrier protein which provides a soluble product. One of the possible solubilising carrier proteins is E.coli SlyD, which, according to Hottenrott et al in the Journal of Biological Chemistry, vol. 272, no. 25, 20 June 1997, pages 15697-15701, has homology to peptidyl-prolyl cis/trans-isomerases (PPlase).

WO97/10253 disloses a high-throughput assay for screening for compounds capable of binding to a fusion protein, which consists of a target protein and a FK506-binding protein. Recombinant DNA and protein sequences for several-fusion proteins are also disclosed.

WO00/20606 relates to a polynucleotide encoding a fusion protein which is stable in a cell. The fusion protein comprises a first polypeptide and a second polypeptide which co-precipitates a chaperone.

Ideno et al in Eur. J Biochem vol. 267, no. 11, 2000, pages 3139-3149 describe a short archaeal FKBP from Methanococcus thermolithotrophicus. The constructs are shown to have PPlase activity and also chaperone-like activity.

Maruyama et al in Front Biosci vol. 5, 2000 pages D821 D836 describe a 28.3 kDa FKBP being from a thermphilic acrchaeum which was expressed in E.coli. Its gene product (MbFK) was characterised was shown to suppress the aggregation of chemically unfolded rhodanese and elevated the yield of its refolding.

It has been reported that, in the case of an antibody, when an antibody is expressed in a periplasmic region by adding a signal sequence to its N-terminus, an antibody can be expressed in a soluble fraction even using Escherichia coli as a host (Glockshuber, Biochemistry 31, 1279-, 1992). However, since a periplasmic region is a very narrow region as compared with cytoplasmic region, an amount of an expressed protein is very small and, even when an expression amount can be increased, a protein becomes an inclusion body. Some trials of expressing an antibody as a soluble entity in a cytoplasm have been reported. A device of increasing a yield of a soluble type by preventing of formation of an inclusion body from a recombinant antibody by coexpressing molecular chaperone which is involved in re-folding of a protein and an antibody gene in a cytoplasm, and a method of using a thioredoxin reductase-deficient strain as a host Escherichia coli have been proposed (Japanese Kokai Publication Hei-9-220092; Ploba, Gene 159, 203-, 1995). However, although these methods can afford a soluble type antibody, a yield of the antibody is very low as around 1 mg/1 L medium (Levy, Protein Expression and Purification 23, 338-, 2001), and a method having a further better production efficacy is required.

On the other hand, since a membrane protein has nature of being present on the surface of a biomembrane or embedded in the interior of the biomembrane, and the protein has a high content of hydrophobic amino acids, it is known that the protein is often expressed as an inclusion body when expressed as a recombinant protein in the absence of a membrane. When toxicity to a cell is exhibited, it does not lead to expression in many cases. When a recombinant type of a membrane protein is obtained, the conventional means is to use a eukaryotic cell such as yeast cell and an animal cell and express the recombinant type in its membrane fraction. However, an expression amount is small, and cost and labor are necessary upon culturing expression and, therefore, a simpler expression method is demanded.

### SUMMARY OF THE INVENTION

In view of the aforementioned circumstances, an object of the present invention is to provide an expression vector, a host, a fused protein, a protein, a process for producing a fused protein, and a process for producing a protein, which can prevent formation of an unactive abnormal protein at production of a recombinant protein, and can produce a desired protein as a natural type, that is, a soluble type at a large amount and effectively.

That is, the present invention is an expression vector, which comprises: (a) a first coding region encoding peptidyl-prolyl cis-trans isomerase (PPlase) having molecular chaperone activity; and (b) a region having at least one restriction enzyme site in which a second coding region encoding a desired protein can be inserted; wherein the PPIase having molecular chaperone activity is archaebacterial FKBP-type PPIase, or comprises an IF domain and/or C-terminal domain of archaebacterial FKBP-type PPlase; and
the first coding region is operatively linked to a promoter, and the restriction enzyme site is in the same reading frame as the first coding region, and is downstream of the first coding region.

lida et al in GENE Vol. 256, No. 1-2, 2000 pages 319-326 disclose plasmid pGEX-HcFK-1 which expresses a fusion protein of glutathione S-transferase (GST) and FKBP-type PPlase. In the expression vector according to this invention, the location of the gene encoding PPIase having molecular chaperone activity is different to that in this reference.

The expression vector of the present invention preferably has a region being between the first coding region and the region having at least one restriction enzyme site in which the second coding region can be inserted, and being translated in the same reading frame to be a protease digestion site.

It is preferable that the express vector of the present invention has an inserted second coding region encoding a protein.

In the expression vector of the present invention, it is preferable that a polypeptide having a molecular chaperone activity is PPIase having molecular chaperone activity.

Examples of the PPIase having chaperone activity also include PPIase comprising an IF domain and/or a C-terminal domain of archaebacterial FKBP-type PPIase.

In the expression vector of the present invention, it is preferable that the second coding region has a nucleotide sequence encoding a monoclonal antibody, or a nucleotide sequence encoding a membrane protein.

A host containing the expression vector of the present invention is one of the present inventions.

It is preferable that the host of the present invention is Escherichia coli.

A fused protein comprising a polypeptide having the molecular chaperone activity obtainable from expression of the expression vector of the invention is also one of the present inventions.

It is preferable that the fused protein of the present invention comprises a protease digestion site.

A process for producing the fused protein of the present invention is one of the present inventions.

In the process for producing a fused protein of the present invention, it is preferable that a host containing the expression vector of the present invention is cultured under condition of expression of the expression vector, and the fused protein is expressed in a cytoplasm, or a region which is transcribed and translated to become a signal sequence is disposed at a 5'-terminus of a first coding region of the expression vector of the present invention or 5'-terminus of a second coding region, a host containing the resulting expression vector is cultured under condition of expression of the expression vector, and the fused protein is expressed in a periplasm or a medium, or the expression vector of the present invention is made to express the fused protein in a cell-free translation system.

In the process for producing a fused protein of the present invention, it is preferable that a fused protein is adsorbed onto a carrier harboring macrolide, cyclosporin, juglone or an analogous compound which inhibits PPIase activity, and then the carrier is recovered.

A process for producing a protein encoded by the second coding region, which comprises digesting a fused protein obtained by the process for producing a fused protein of the present invention with a protease which digests a protease digestion site, is also one of the present inventions.

### BRIEF DESCRIPTION OF THE DRAWING(S)

Fig. 1 is a view showing a gene arrangement of the vector TcFKfusion2 for making a fused protein with Thermococcus sp. KS-1 short type FKBP-type PPIase.
Fig. 2 is a view showing expression of TcFKBP18 in the case where TcFKfusion2 is used.
Fig. 3 is a view showing an electrophoretic pattern of a host protein.
Fig. 4 is a view showing expression of a fused protein of mouse anti-hen egg white lysozyme (HEL) Fab antibody fragment and TcFKBP18.
Fig. 5 is a view showing expression of mouse anti-hen egg white lysozyme (HEL) Fab antibody fragment alone.
Fig. 6 is a view showing expression of a fused protein of mouse anti-hen egg white lysozyme (HEL) scFv fragment and TcFKBP18.
Fig. 7 is a view showing a fused protein of purified mouse anti-HEL scFv and TcFKBP18, and results of treatment of the fused protein with thrombin.
Fig. 8 is a view showing activity of mouse anti-HEL scFv obtained as a result of expression, by an ELISA method.

### DETAILED DESCRIPTION OF THE INVENTION

The present invention will be described in detail below.

In the present invention, "operatively linked to a promoter" means that a first coding region is linked to a promoter so that a polypeptide having molecular chaperone activity is normally transcribed, or a second coding region is linked to a promoter so that a desired protein is normally transcribed.

In the present invention, "PPIase having molecular chaperone activity" includes those having substantially equivalent function. That is, substantially equivalent polypeptides, polypeptides comprising at least a part of them, and those in which a part of amino acids are altered to other amino acids, are included.

Further, in the present invention, a "domain" includes a domain having substantially equivalent function.

The expression vector of the present invention comprises (a) a first coding region encoding a polypeptide having molecular chaperone activity.

The molecular chaperone activity means activity of re-folding a denatured protein into an original natural type, or activity of inhibiting irreversible aggregation of a denatured protein. For example, rhodanese, citrate synthetase, malate dehydrogenase, glucose-6-phosphate dehydrogenase and the like are used as a model enzyme (Kawata, Bioscience and Industry 56, 593-, 1998), these are denaturation-treated with a protein denaturing agent such as 6M guanidine hydrochloride, thereafter, molecular chaperone activity of a test substrate can be assessed by a rate of regeneration of a denatured protein which is initiated upon dilution of a denaturing agent with a buffer containing the test substance, and a rate of inhibiting aggregation of a denatured protein. Examples of a method of assessing a rate of regeneration of a denatured protein include a method of Horowitz et al. (Horowitz, Methods Mol. Biol.40, 361-, 1995) in the case of rhodanese. Examples of a method of assessing inhibition of aggregation of a denatured protein include a method of Taguchi et al. (Taguchi, J. Biol. Chem.269, 8529-, 1994).

PPIase (Peptidyl-prolyl cis-trans isomerase) having the molecular chaperone activity is one of protein folding factors involved in folding of a protein, and has activity of catalyzing a cis trans isomerization reaction of a N-terminal peptidyl bond of a proline residue (PPIase activity) among amino acids in a target protein during protein folding in a cell.

PPIase having the molecular chaperone activity is classified into three types, FK506 Binding Protein type (FKBP type), cyclophilin type and parvulin type based on sensitivity to it inhibitor. FKBP type PPIase is PPIase, activity of which is inhibited by FK506 which is one of immunological inhibitors, and homologues thereof. Cyclophilin type PPIase is PPIase having sensitivity to cyclosporine which is another immunological inhibitor, or homologues thereof. On the other hand, parvulin type PPIase is PPIase exhibiting no sensitivity to those immunological inhibitors, and activity of which is inhibited by juglone, or homologues thereof. These three kinds of PPIases have little homology on an amino acid primary sequence.

As PPIase having the molecular chaperone activity, archaebacterial FKBP-type PPIase is used.

Regarding function of the archaebacterial FKBP-type PPIase, interestingly, it has been found that it has not only PPIase activity, but also molecular chaperone activity of inhibiting irreversible aggregation of a protein and, at the same time, promoting re-folding of a denatured protein (Furutani, Biochemistiry 39, 453-, 2000; Ideno, Eur. J. Biochem. 267, 3139-, 2000; Ideno, Biochem. J. 357, 465-, 2001; Ideno, Appl. Env. Microbiol. 68, 464-, 2002). Molecular chaperone activity is originally activity which was found in protein folding system of chaperonin known one of molecular chaperone, and DnaK/DnaJ/GrpE system. These exerts function of supporting so that a polypeptide biosynthesized in a cell is folded into a correct form. Thereupon, hydrolysis of a high energy substance such as ATP is required. The archaebacterial FKBP-type PPIase is excellent in that a reaction of hydrolyzing the high energy substance is not required upon manifestation of the molecular chaperone activity.

The archaebacterial FKBP-type PPIase can be roughly classified into two kinds based on a difference in a molecular weight. One is a short type having a molecular weight of around 16 to 18kDa, and the other is along type of around 26 to 33kDa. As the archaebacterial FKBP-type PPIase used in the present invention, any of short type and long type FKBP-type PPIases may be used. However, generally, in view of two points that a short type tends to have stronger molecular chaperone activity, and as a molecular weight of a protein grows larger, an amount of expression of its recombinant protein tends to be decreased, the short type archaebacterial FKBP-type PPIase is preferable in the present invention. A width of the aforementioned molecular weight is a molecular weight width of the previously found PPIase, the archaebacterial FKBP-type PPIase in the present invention is not limited to this molecular weight width, any may be used as long as it belongs to substantially the same group.

The archaebacterial FKBP-type PPIase is not particularly limited, but may be derived from any archaebacteria. For example, among the previously found archaebacterial FKBP-type PPIases, examples of a short type include PPIases derived from thermophilic and hyperthermophilic archaebacteria such as Methanococcus thermolithotrophicus, Thermococcus sp. KS-1, and Methanococcus jannaschii, and derived from mesophilic archaebacteria such as Methanosarcina mazei, Methanosarcina acetivorans, and Methanosarcina barkeri (Maruyama, Front.Biosci 5, 821-, 2000). On the other hand, as a result of genome analysis and other analysis, a long type has been found on a genome of almost all archaebacteria. Examples include PPIases derived from thermophilic and hyperthermophilic archaebacteria such as Pyrococcus horikoshii, Aeropyrum pernix, Sulfolobus solfataricus, Methanococcus jannaschii, Archaeoglobus fulgidus, Methanobacterium autotrophicum, Thermoplasma acidophilum, and derived from mesophilic archaebacteria such as Halobacterium cutirubrum (Maruyama, Front. Biosci 5, 821-, 2000). Inter alia, PPIase derived from mesophilic archaebacteria is preferable. As one example of the aforementioned long type archaebacterial FKBP-type PPIase, an amino acid sequence derived from Pyrococcus horikoshii is shown in SEQ ID NO: 1 and, as one example of the aforementioned short type archaebacterial FKBP-type PPIase, an amino acid sequence derived from Methanococcus jannaschaii is shown in SEQ ID NO: 2.

The archaebacterial FKBP-type PPIase has a FKBP domain involved in PPIase activity and binding with FK506, and an IF domain, and the long type archaebacterial FKBP-type PPIase has further a C-terminal domain (Maruyama and Furutani, Front Biosci. 1, D821-, 2000). The IF domain (Insert in the flap; Suzuki, J. Mol. Biol.328, 1149-, 2003) comprises about 100 amino acids inserted in an amino acid sequence constituting a FKBP domain, on an amino acid primary sequence, and forms a characteristic high order structure forming a domain structure (Maruyama and Furutani, Front Biosci. 1, D821-, 2000; Insert in the flap; Suzuki, J. Mol. Biol. 328, 1149-, 2003). It has been revealed that a FK506 binding region of a FKBP domain and an IF domain are involved in molecular chaperone activity of the short type archaebacterial FKBP-type PPIase (Furutani, Biochemistry 39, 2822-, 2000; Ideno, Biochem. J. 357, 465-, 2001). In addition, it has been revealed that, regarding the long type archaebacterial FKBP-type PPIase, a C-terminal domain together with the aforementioned two domains is involved in the molecular chaperone activity (IDENO, Eur. J. Biochem. 267, 3139-, 2000). In the present invention, PPIase comprising an IF domain and/or a C-terminal domain of archaebacterial FKBP-type PPIase can be used as PPIase having the molecular chaperone activity. For example, chimera PPIase in which an IF domain and a C-terminal domain are introduced by protein engineering into human FKBP12 which is PPIase originally having no molecular chaperone activity can be used as PPIase having the molecular chaperone activity. As an IF domain of the archaebacterial FKBP-type PPIase, a region of 78^{th} proline to 140^{th} tyrosine, in SEQ ID NO: 1, and a region of 78^{th} proline to 141^{st} glutamic acid in SEQ ID NO: 2 corresponds to IF domain respectively (IDENO, Eur. J. Biochem. 267, 3139-, 2000). On the other hand, as a C-terminal domain of the archaebacterial FKBP-type PPIase, a region of 157^{th} isoleucine to a C-terminus in SEQ ID NO: 1 corresponds to a C-terminal domain (IDENO, Eur. J. Biochem. 267, 3139-, 2000). Homology of each domain can be determined by using multiple alignment software such as ClustalW.

The expression vector of the present invention comprises (b) a region having at least one restriction enzyme site in which a second coding region encoding a protein can be inserted.

The second coding region is a region having a nucleotide sequence encoding a desired protein which is to be expressed using the expression vector of the present invention.

A second coding region used in the present invention is not particularly limited, and examples include a nucleotide sequence encoding an antibody such as a monoclonal antibody and nucleotide sequences encoding a membrane protein.

The antibody may be an antibody derived from any animal species, and a polypeptide in which a full length antibody, a fragment thereof, or two or more fragments such as Fab, Single chain Fv(scFv) and the like are linked thereto with a linker peptide is included in the antibody. The antibody may be any subclass.

An antibody is a huge molecule having a molecular weight exceeding 100 thousands, is widely used as an analysis reagent or an in vitro diagnostic utilizing function of specifically binding to a specified antigenic substance, and has high industrial utilization value. A part contributing to binding of an antibody molecule and an antigenic substance is called V region (variable region), and is composed of a V region of a heavy chain and a V region of a light chain. As a method of obtaining an antibody to a specified antigen, a method of immunologically sensitizing an experimental animal such as rat and rabbit with an antigenic substance and obtaining an antibody (polyclonal antibody) containing in a serum, and a method of obtaining a monoclonal antibody described below are general.

A monoclonal antibody is an antibody which is produced by an antibody-producing cell of a single clone, and its characteristic is that a primary structure is uniform. A monoclonal antibody can be easily produced by establishment of hybridoma technique by Kohler and Milstein. In this method, first, a predetermined antigenic substance is administered to an experimental animal such as mouse to perform immunological sensitization. Then, a spleen cell which has acquired antibody-producing ability to the antigenic substance is taken out from spleen of an immunologically sensitized animal, and this is fused with an appropriate tumor cell such as myeloma to make a hybridoma. Then, by screening using an appropriate immunological analysis method such as ELISA, a hybridoma producing a desired antibody is selected. Thereafter, by cloning using a limiting dilution method, a hybridoma strain producing a desired monoclonal antibody is established. After the thus established hybridoma is cultured in an appropriate medium, a desired monoclonal antibody is obtained by separating a medium including the metabolite using chromatography etc. However, since these methods utilize an in vivo living body reaction of immunological sensitization of an animal, they inevitably require interposition of an experimental animal. Therefore, an experimental animal must be bred and maintained, troublesome labor is necessary and, at the same time, much cost is necessary. In addition, by this method, a monoclonal antibody to all antigenic substances can not be necessarily produced, and trial and error element is included. Recently, it has become possible to express scFv in which only V regions of a heavy chain and a light chain of an antibody are linked via an appropriate linker, or a Fab part of an antibody, on a superficial layer of Escherichia coli. A method of making an library of an antibody gene by randomly amplifying antibody genes using PCR, making present extracellularly, and screening a member having affinity with a specified antibody from these libraries is being developed (Kumatani et al., Protein·Nucleic Acid·Enzyme 43, 159-, 1998). When the antibody gene obtained by screening is expressed by Escherichia coli or the like, an antibody to a desired antigen can be made without using an experimental animal. However, for example, when an antibody gene is expressed in Escherichia coli at a large amount, almost all was expressed as an insoluble inclusion body, and active type can not be obtained as described above.

To the contrary, when a nucleotide sequence encoding a monoclonal antibody as a second coding region is inserted into the expression vector of the present invention, it becomes possible to easily obtain an active type (soluble type) product of the antibody obtained by screening.

The aforementioned membrane protein is not particularly limited, and examples include a receptor for a physiologically active substance. Since the aforementioned receptor for a physiologically active substance selectively responds to extracellular various substances, and transmits a variety of signals into a cell, this is paid much attention because clarification of the function directly leads to drug generation. These membrane proteins form a structurally well conserved family, and are roughly classified into three of ion channel intrinsic type, tyrosine kinase type and G protein-coupled type. The ion channel intrinsic type is a type such that, when a ligand binds to a receptor, an ion channel present in the receptor itself is opened, Na⁺ and Ca²⁺ are transferred into a cell utilizing an ion gradient between outside and inside a cell. The tyrosine kinase type converts a binding of a ligand into increased in phosphorylation activity to cause a series of cascades, and thereby amplifying a signal. In the G protein-coupled type, a receptor itself has not an ion channel and enzymatic activity, but transmits information by binding of a ligand into a cell via G protein. Many medicaments targeting a membrane receptor protein have been developed, and many of them target a G protein-coupled receptor (GPCR). Therefore, by specifying an endogenous ligand of GPCR and further clarifying its function and structure, possible rapid development of medicaments can be expected. For crystallization and deuteration for these ligand screening and structural analysis, development of large amount expression technique of GPCR is indispensable, but the previous development of GPCR is impossible in Escherichia coli and yeast. Currently, various analyses are performed using a minor amount of a sample expressed mainly in an animal cultured cell such as CHO, COS-7 and HEK.

To the contrary, when a nucleotide sequence encoding a membrane protein as a second coding region is inserted into the expression vector of the present invention, a recombinant protein can be produced at a low cost and a large amount.

The aforementioned restriction enzyme site is also called multicloning site. A region having the restriction enzyme site is a region in which a gene encoding a desired protein is inserted as a second coding region.

In the expression vector of the present invention (1) the first coding region is operatively linked to a promoter, and the restriction enzyme sites are in the same reading frame as that of the first coding region, and are downstream of the first coding region, or (2) the second coding region in which the restriction enzyme sites are inserted is disposed so that the region is operatively linked to a promoter, and the first coding region is in the same reading flame as that of the second coding region, and is downstream of the second coding region.

The promoter is not particularly limited, and examples include a Plac promoter, a Ptac promoter, a xylA promoter, an AraB promoter, a lambda promoter, a T7 promoter, a ga11/ga110 promoter, a nmtl promoter, a polyhedrin promoter, and a mouse metallothionein promoter.

In the expression vector of the present invention, by inserting a second coding region encoding a desired protein into the restriction enzyme sites to obtain an expression vector in which a second coding region is inserted, and expressing this expression vector, (1) when the first coding region is operatively linked to a promoter, and the restriction enzyme sites are in same reading frame as that of the first coding region, and are downstream of the first coding region, a first coding region and, subsequent a second region are translated by the promoter, and on the other hand, (2) when the second coding region in which the restriction enzyme sites are inserted is disposed such that it is operatively linked to a promoter, and the first coding region is in the same reading frame as that of the second coding region, and is downstream of the second coding region, a second coding region and a subsequent a first coding region are translated by the promoter and, in both cases, a desired protein encoded by a second coding region is expressed as a fused protein with a polypeptide having molecular chaperone activity.

The expression vector of the present invention may have a region which is between a region having at least one restriction enzyme site in which the first coding region and the second coding region can be inserted, and which is translated in the same reading frame to become a protease digestion site.

The aforementioned protease digestion site is to be a peptide linker for linking a polypeptide described above and a protein described below, in a fused protein in which a polypeptide having molecular chaperone activity encoded by first coding region and a protein encoded by a second coding region are bound, which is obtained by expression of an expression vector in which a second coding region is inserted into the expression vector of the present invention. Since the resulting fused protein has a protease digestion site, a protease is acted to easily digest the fused protein, whereby, a polypeptide having molecular chaperone activity encoded by a first coding region and a protein encoded by a second coding region are separated, and a desired protein encoded by a second coding region can be obtained.

The protease is not particularly limited, and examples include thrombin, factor Xa, and precision protease. These proteases are sold by Pharmacia-Biotech. Alternatively, a desired protein can be separated utilizing self protein splicing function of intein.

A length of a nucleotide sequence encoding the protease digestion site is not particularly limited, and around 15 to 90 basis is preferable, and it is preferable that many nucleotide sequences which are translated to be a neutral amino acid such as glycine and serine are contained.

The expression vector of the present invention may comprise other known nucleotide sequences. The other known nucleotide sequences are not particularly limited, and examples include a stability leader sequence which imparts stability to an expression product, a signal sequence which imparts secretion of an expression product, and a marking sequence which can impart phenotype selection in a transformed host such as a neomycin resistance gene, a kanamycin resistance gene, a chloramphenicol resistance gene, an ampicillin resistance gene, and a hygromycin resistance gene

The expression vector of the present invention may be designed into a form that the resulting fused protein binds to a carrier for its immobilization via an appropriate ligand. Thereby, after expression, purification can be simplified. For example, in the case where PPIase is used as a polypeptide having molecular chaperone activity, purification of a fused protein can be simplified by using a carrier harboring FK506, rapamycin or an analogous compound in the case of FKBP-type PPIase, by using a carrier harboring cyclosporin or its analogous compound in the case of cyclophilin-type PPIase, or by using a carrier harboring juglone or its analogous compound in the case of parvulin-type PPIase.

In addition, when the expression vector of the present invention is designed so that it has a tag of about six histidine residues on a N-terminal side of a polypeptide having the molecular chaperone activity, since the resulting fused protein binds to a carrier chelated with a metal such as nickel via a histidine residue, a protein derived from a host and a fused protein can be simply separated using the carrier. Further, by acting a protease on a fused protein bound to the carrier, the aforementioned protease digestion site is digested, and only a desired protein can be simply freed from a carrier.

When eluted with imidazole, a fused protein, as it is, may be freed from a carrier without acting a protease. In addition to the aforementioned histidine tag, a method of purification by affinity chromatography with a glutathione resin using glutathione-s-transferase or a part thereof as a tag, and a method of purification with a maltose resin using a maltose binding protein or a part thereof as a tag may be used. Besides, affinity with an antibody may be used. The aforementioned various tags may be designed on either of a N-terminal side and a C-terminal side of a fused protein, or may be designed on both sides. As these genetic manipulation methods, and affinity purification method, methods known to a person skilled in the art may be used.

By incorporating a gene encoding a desired protein as a second coding region in the expression vector of the present invention to express the gene, a fused protein of a polypeptide having molecular chaperone activity encoded by a first coding region and a protein encoded by a second coding region is obtained. Such the fused protein comprising a polypeptide having the molecular chaperone activity and a protein encoded by a second coding region is also one of the present inventions. Further, when a linker peptide comprising a protease digestion site is introduced between both of them, a desired protein can be easily separated from a fused protein by digesting the resulting fused protein with a protease. For this reason, it is preferable that the fused protein of the present invention comprises a protease digestion site.

The expression vector of the present invention is introduced into a host, and serves for expression of the desired protein. Such the host containing the expression vector of the present invention is one of the present inventions.

The host is not particularly limited, and examples include a prokaryote such as bacterium, yeast, fungus, plant, insect cell, and mammal cell. However, property of a host to be used and that of an expression vector must be adapted. For example, when a fused protein is expressed in a mammal cell system, it is preferable that an expression vector uses a promoter isolated from a genome of a mammal cell such as a mouse metallothionein promoter, or a promoter isolated from virus growing in a mammal cell such as a baculovirus promoter, and a vaccinia virus 7.5K promoter.

As the host, inter alia, a prokaryote such as Escherichia coli is preferable.

A method of introducing the expression vector of the present invention into a host is not particularly limited, and the known various methods can be used, and examples of transfection include a calcium phosphate precipitation method, electroporation, liposome fusion, nuclear injection, and virus or phage infection.

A large amount of a fused protein can be obtained by introducing the expression vector of the present invention into an appropriate host, and culturing the host under appropriate condition to express. Such the process for producing a fused protein of the present invention is also one of the present inventions.

In the process for producing a fused protein of the present invention, it is preferable that a host containing the expression vector of the present invention is cultured under condition of expression of the expression vector to express the fused protein in a cytoplasm, a host containing an expression vector obtained by providing a region which is transcribed and translated to be a signal sequence at a 5'-terminus of a first coding region of the expression vector of the present invention or 5'-terminus of the second coding region is cultured under condition of expression of the expression vector to express the fused protein in a periplasm or a medium, or the expression vector of the present invention is made to express the fused protein in a cell-free translation system.

When a Gram-negative bacterium is used as a host, expression of a fused protein may be expression in a cytoplasm, or in a periplasm or a medium. By providing a signal sequence which is transcribed and translated at a 5'-terminus of a first coding region or 5'-terminus of a second coding region in the expression vector of the present invention, the fused protein can be secreted and expressed in a periplasm or a medium. When the fused protein is expressed in a periplasm, expression property can be improved by using a polypeptide which is originally present in a membrane in a cell as a polypeptide encoded by a first coding region. Examples of the polypeptide which is originally present in a membrane in a cell include FkpA-type PPIase which is FKBP-type PPIase, and SurA-type PPIase which is parvulin-type PPIase. These PPIases are a protein which is originally present in a periplasm of a Gram-negative bacterium, and is involved in folding of a protein.

When a protein encoded by the second coding region is a membrane protein or an antibody, since these proteins are a protein which is originally expressed extracellularly, expression property is improved by expression by fusion with the FkpA-type PPIase or SurA-type PPIase.

When the expression vector of the present invention is made to express the fused protein in a cell-free translation system, the fused protein of the present invention is expressed as a soluble protein in a cell-free translation system using bacteria or eukaryote extract (Spirin, A.S., 1991, Science 11, 2656-2664:Falcone, D. et.al., 1991, Mol. Cell. Biol. 11, 2656-2664) without using a host cell.

In the process for producing the fused protein of the present invention, it is preferable that after a fused protein-is adsorbed onto a carrier harboring macrolide, cyclosporin, juglone or its analogous compound which inhibits PPIase, the carrier is recovered.

Binding property between PPIase having the molecular chaperone activity and the aforementioned inhibitor is strong, and an expressed fused protein can be purified utilizing this affinity. For example, when a bead harboring macrolide such as FK506 on an agarose gel carrier is used, a fused protein with FKBP-type PPIase can be specifically bound.

By digesting a fused protein obtained by the process for producing a fused protein of the present invention with a protease which digests a protease digestion site, a desired protein can be obtained. Such the process for producing a protein encoded by the second coding region is also one of the present inventions.

According to the present invention, by linking a desired protein and a polypeptide having molecular chaperone activity with a peptide linker to express as a fused protein, a hardly expressible protein which is originally expressed as an abnormal type can be expressed as a natural type soluble form at a large amount, and its productivity can be improved considerably. In addition, when a desired protein is an antibody, according to the present invention, since it becomes possible to simply produce a recombinant-type antibody having function without using an experimental animal, it becomes possible to produce a highly functional antibody at a large amount by fusion with other protein or peptide.

### BEST MODE FOR CARRYING OUT THE INVENTION

The following Examples further illustrate the present invention in detail.

### (Example 1) Construction of expression vector for fusion with the short type FKBP-type PPIase (TcFKBP18), which is derived from hyperthermophilic archaebacterium Thermococcus sp. KS-1

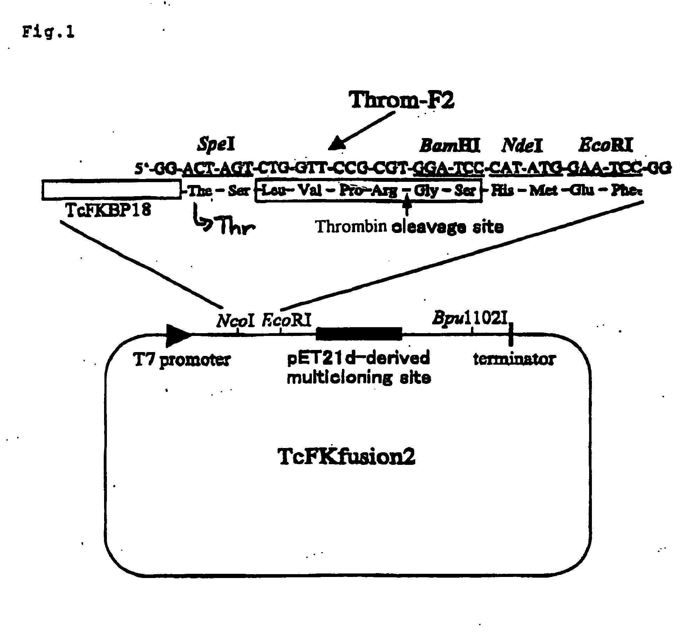

Using an expression plasmid pEFE1-3 (Iida, Gene 222, 249-, 1998) of TcFKBP18 (Ideno, Biochem. J. 357, 465-, 2001) having molecular chaperone activity as a template, the TcFKBP18 gene fragment was amplified by a PCR method. By using TcFu-F1 and TcFu-R2 shown in Table 1 as a primer set for PCR, a restriction enzyme site was provided on both sides of the amplification product. On the other hand, as a nucleotide sequence encoding a linker for cutting TcFKBP18 fused protein into TcFKBP18 and a desired protein with a protease, Throm-F2 and its complementary chain were designed. Throm-F2 has a SpeI site on its 5' side and an EcoRI site on its 3' side, respectively (Fig. 1). Since Throm-F2 has a BamHI site and a NdeI site downstream of a DNA sequence of a thrombin cleavage site, a fused protein with TcFKBP18 can be obtained by introducing a gene fragment of a desired protein utilizing these restriction enzyme sites (Fig. 1).

A gene fragment of the TcFKBP18 and a DNA fragment encoding a thrombin cleavage site were treated with restriction enzymes respectively, and ligated to a pET21d plasmid DNA which had been treated with NcoI/EcoRI in advance (manufactured by Novagen), in an order of TcFKBP18 gene-Thermo-F2. The resulting plasmid for expressing TcFKBP18 fused protein was designated TcFKfusion2.

**Table 1**

| Abbrev. | Sequence | Restriction site |
|---|---|---|
| Tcfu-F1 | 5'-GGCCATGGGAAAAGTTGAAGCTGGTGAT-3' | *Nco* I |
| Tcfu-R2 | 5*'-*CCACTAGTAGCTTCTGAGTCCTCTTC-3' | *Spe* I |
| TF-F1 | 5'-GGCCATGGGCCAAGTTTCAGTTGAAACC-3' | *Nco* I |
| TF-R1 | 5'-CCACTAGTCGCCTGCTGGTTCATCAGCT-3' | *Spe* I |
| FK52-F1 | 5'-GGCCATGGGCACAGCCGAGGAGATGAA-3' | *Nco* I |
| FK52-R1 | 5'-CCACTAGTTGCTTCTGTCTCCACCTGA-3' | *Spe* I |
| CP40-F1 | 5'-GGCCATGGGCTCGCACCCGTCCCC-3' | *Nco* I |
| CP40-R1 | 5'-CCACTAGTAGCAAACATTTTTGCATATACTG-3' | *Spe* I |
| FKPA-F1 | 5'-GGCCATGGCAAATCACTGTITAAAGTAACGC-3' | *Nco* I |
| FKPA-R1 | 5'-CCACTAGTTTTTTTAGCAGAGTCTGCGGC-3' | *Spe* I |
| SUR-F1 | 5'-GGCCATGGGCAAGAACTGGAAAACGCTG-3' | *Nco* I |
| SUR-R1 | 5'-CCACTAGTGTTGCTCAGGATTTTAACGTA-3' | *Spe* I |
| SCF-F3 | 5 '-ATCATATGAAATACCTATTGCCTACG-3' | *Nde* I |
| SCF-R3 | 5'-ATGCGGCCGCTATTACTCCAGCTTGGTCCCTC-3' | *Not* I |

| | | |
|---|---|---|
| Underline:each restriction enzyme site | | |

### (Example 2) Expression of TcFKBP18 using TcFKfusion2

E. coli BL21 (DE3) strain was transformed with TcFKfusion2 prepared in Example 1. 700 mL of a 2 x YT medium (Yeast Extract 16 g/L, BACTO TRYPTON 20 g/L, NaCl 5 g/L, ampicillin 100 µg/mL, pH 7.5) was placed into a 2L Erlenmeyer flask, and this was inoculated with 2 to 3 platinum loops of recombinant Escherichia coli. After rotation culturing (110 rpm) at 35°C for 24 hours, cells were recovered by centrifugation (10000 rpm x 10 min). The resulting cells were suspended in 20 mL of a 25 mM HEPES buffer (pH 6.8) containing 1 mM EDTA, and freezing-stored at -20°C.

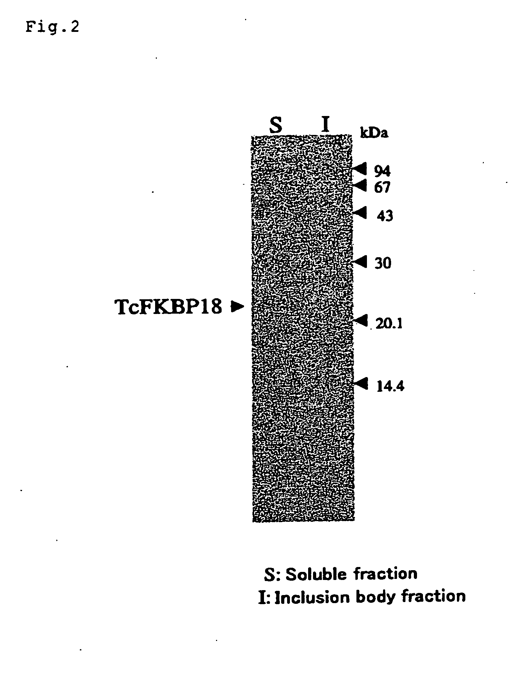

The resulting cells were disrupted by sonication, and centrifuged to separate into the supernatant (soluble fraction) and the precipitate part (precipitate fraction). In order to further purify to an inclusion body fraction, the precipitate fraction was suspended in a 25 mM HEPES/1 mM EDTA buffer (pH 6.8) containing 4% Triton X-100, and thereafter the materials were reacted for 30 minutes to solubilize membrane components, and precipitated inclusion body components were recovered by centrifugation. This procedure was repeated two times, and the resulting precipitated part was regarded as an inclusion body fraction. 10 µg of a soluble fraction and a volume of an inclusion body fraction corresponding it were subjected to 16% SDS-PAGE. As a result, a band corresponding to TcFKBP18 was seen only in a soluble fraction. A band was detected at a position corresponding to a higher apparent molecular weight than a position at which TcFKBP18 is originally detected (Fig. 2). The reason can be considered as follows: Since a termination codon is not present at a 3' terminus of a structural gene of TcFKBP18, and a multicloning site is present there, the translation product is connected to a C-terminus of TcFKBP18.

### (Example 3) Expression of fused protein composed of TcFKBP18 and mouse anti-hen egg white lysozyme (HEL)Fab antibody fragment (D1.3)

An expression plasmid pEHELFab-1(Ideno, Appl. Env. Microbiol.68, 464-, 2002) for a mouse anti-HEL Fab antibody fragment was treated with NdeI/Bpul102I, and an anti-HEL Fab antibody fragment gene fragment was purified by an electrophoresis method using an agarose gel. This DNA fragment was ligated to TcFKfusion2 which had been treated with NdeI/Bpul102I in advance. When the plasmid obtained as this result was expressed, a heavy chain part of the Fab was expressed as a fused protein with TcFKBP18, and it was found that a light chain part is expressed alone not as a fused protein. The resulting plasmid was introduced into Escherichia coli in the same method as that of Example 2, and a transformant was obtained. This bacterium was cultured and recovered in the same manner as that of Example 2, and freezing-stored at -20°C.

The resulting cells were disrupted by sonication, and centrifuged to separate into the supernatant (soluble fraction) and the precipitate part (precipitate fraction), which were subjected to SDS-PAGE in the same manner as that of Example 2. SDS-PAGE gel was stained with Coomassie Brilliant Blue (CBB) and the expressed Fab was specifically detected by a Western blotting method using a rabbit anti-D1.3 antibody as a primary antibody.

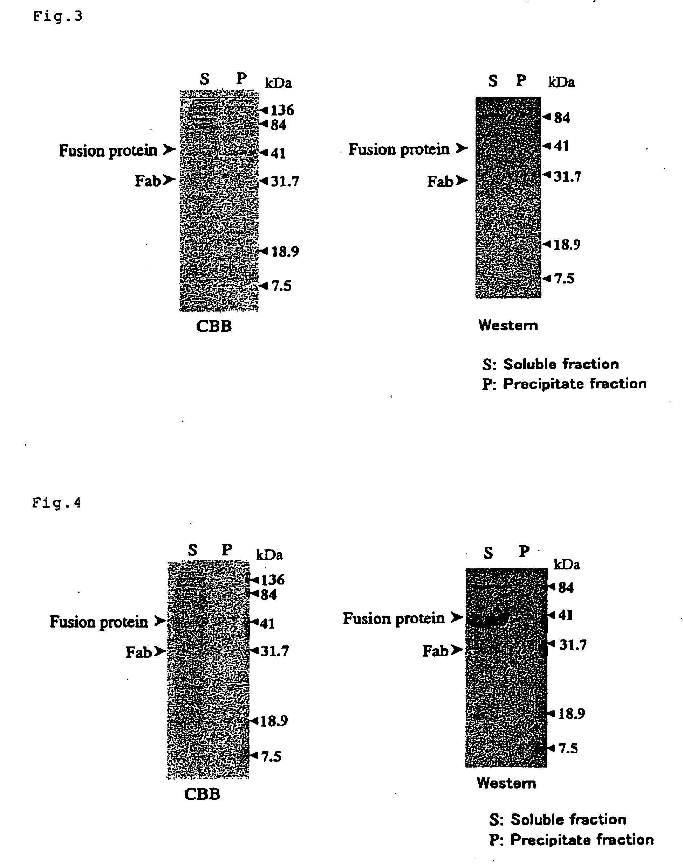

A band corresponding to a fused protein of Fab and TcFKBP18 was not seen in a soluble fraction and a precipitate fraction of Escherichia coli which is a host bacterium, in detection either of CBB staining or Western blotting (Fig. 3). On the other hand, when expressed as a fused protein with TcFKBP18, it was shown that a heavy chain part of Fab is expressed as a major band in a soluble fraction as a form fused with TcFKBP18, in CBB staining, and it was revealed that Fab is assuredly expressed at a large amount also in Western blotting (Fig. 4). In CBB staining, a band density of a fused protein expressed in a soluble fraction was measured with a densitometer and, as a result, the density was about 10% of all soluble proteins derived from Escherichia coli. To the contrary, a band corresponding to a light chain part of Fab was not detected. From the result of Western blotting, it was considered that a light chain of Fab is degraded with a host protease (Fig. 4).

### (Comparative Example 1) Expression of mouse anti-HEL Fab antibody fragment alone

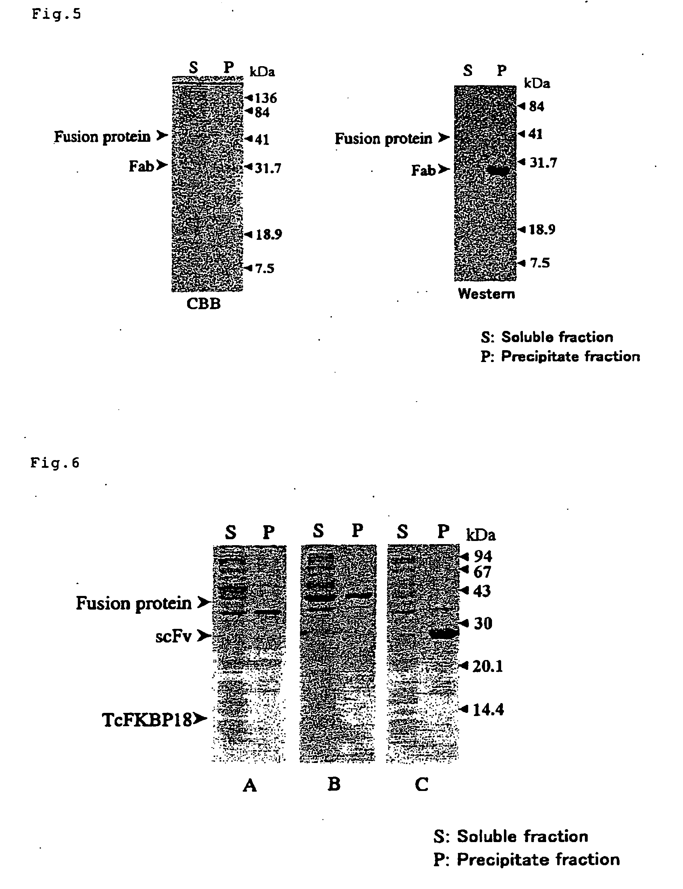

An expression plasmid pEHELFab-1 for mouse anti-HEL Fab antibody fragment was introduced into Escherichia coli in the same manner as that of Example 2, and evaluated with SDS-PAGE. As a result of CBB staining and Western blotting, it was confirmed that a Fab gene alone is not expressed in a soluble fraction, and all is expressed in a precipitate fraction (Fig. 5).

### (Example 4) Expression of fused protein of mouse anti-HEL scFv and TcFKBP18

By PCR using SCF-F3 and SCF-R3 shown in Table 1 as a primer set and using an expression plasmid pAALSC for mouse anti-HEL scFv fragment (Iba et al. 1997, Gene 194, 35-) as a template, a gene of a mouse anti-HEL scFv fragment was amplified. This gene was ligated to a pT7 blue vector by TA cloning, treated with NdeI/NotI, and then ligated again to TcFKfusion2 which had been treated with the same restriction enzymes in advance, to construct a system for expressing a fused protein of TcFKBP18 and scFv. The resulting plasmid was introduced into Escherichia coli in the same manner as that of Example 2, and a transformant was obtained. This bacterium was cultured and recovered in the same manner as that of Example 2, and freezing-stored at -20°C. The resulting cell solution was subjected to SDS-PAGE in the same manner as that of Example 2, and stained with CBB.

A band corresponding to a fused protein of mouse anti-HEL scFv and TcFKBP18 was not detected in a soluble fraction and a precipitate fraction of Escherichia coli which is a host bacterium, in CBB staining (Fig. 6A). On the other hand, when expressed as a fused protein with TcFKBP18, it was shown that mouse anti-HEL scFv was expressed as a major band in a soluble fraction as a form fused with TcFKBP18, at a large amount (Fig. 6B). In CBB staining, a band density of a fused protein expressed in a soluble fraction was measured with a densitometer and, as a result, the density was about 14% of all soluble proteins derived from Escherichia coli.

### (Comparative Example 2) Expression of mouse anti-HEL scFv alone

The pT7 blue vector comprising a mouse anti-HEL scFv gene obtained in Example 4 was treated with NdeI/NotI, and then ligated again to pET21a (manufactured by Novagen) which had been treated with the same restriction enzymes in advance, whereby, a system for expressing mouse anti-HEL scFv was constructed. The resulting expression plasmid was introduced into Escherichia coli according to the same manner as that of Example 4, and a transformant was obtained. This bacterium was cultured and recovered in the same manner as that of Example 2, and freezing-stored at - 20°C. The resulting cell solution was subjected to SDS-PAGE in the same manner as that of Example 2, and stained with CBB. As a result, it was confirmed that mouse anti-HEL scFv is hardly expressed in a soluble fraction, and almost all is expressed in an insoluble fraction (Fig. 6C).

### (Example 5) Purification of fused protein of mouse anti-HEL scFv and TcFKBP18

By repeating column-purification of the soluble fraction obtained in Example 4 in an order of the following (a) and (b) anion exchange chromatography and gel filtration, a fused protein of mouse anti-HEL scFv and TcFKBP18 was purified to almost single. An amount of the fused protein obtained as the result of purification was about 50 mg per 1 L of a medium.
(a) DEAE Toyopearl column (16 mm x 60 cm; manufactured by Tosoh Corporation)
   Solution A: 25 mM HEPES-KOH buffer (pH 6.8)
   Solution B: 25 mM HEPES-KOH buffer (pH 6.8) containing 0.5 M NaCl
   (0 to 300 min: Solution B 0 to 100% linear gradient, 300 to 420 min: Solution B 100%)
   Flow rate: 1 mL/min
(b) HiLoad 26/60 Superdex 200pg column (26 mm x 60 cm; manufactured by Amersham Pharmacia)
   Eluent: 100 mM sodium phosphate buffer (pH 7.0; containing 0.15 M NaCl)
   Flow rate: 3 mL/min

### (Example 6) Cleavage of fused protein with thrombin

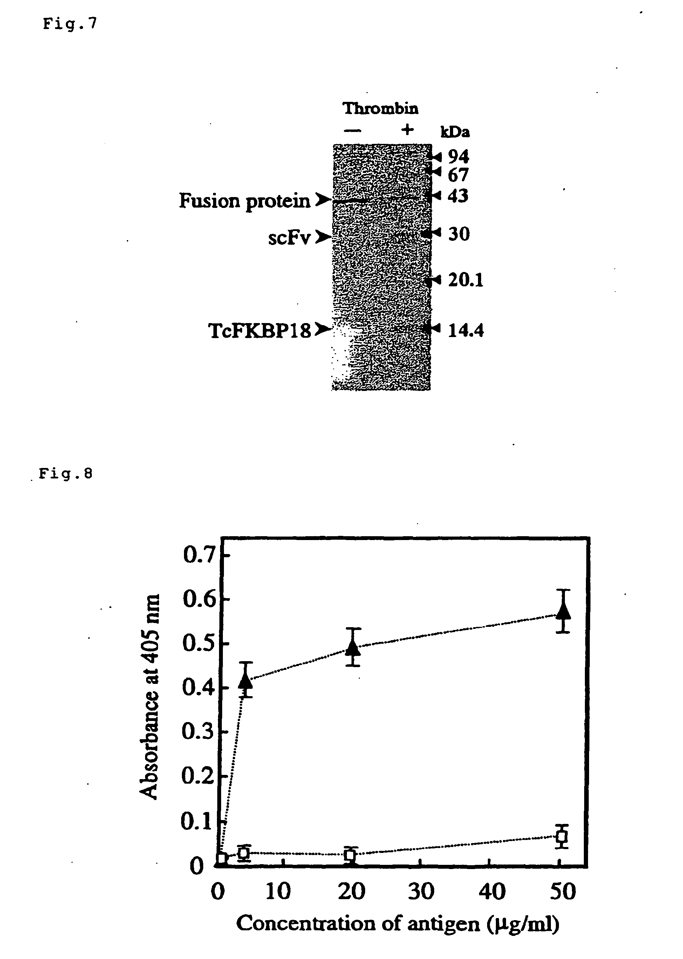

Thrombin was added at 10U per 1 mg of the fused protein purified in Example 5, and treated at 22°C for 16 hours, whereby, a thrombin site of the fused protein was cleaved. As a result of SDS-PAGE, it was confirmed that the fused protein was assuredly cleaved into mouse anti-HEL scFv and TcFKBP18 (Fig. 7).

### (Example 8) Confirmation of function of mouse anti-HEL scFv by ELISA

Function of mouse anti-HEL scFv obtained by expression was assessed by whether it functions as a primary antibody or not in an ELISA method using hen egg white lysozyme as an antigen. That is, 100 µL of a 50 µg/mL hen egg white lysozyme (HEL) solution was added to a 96-well plate, and incubated at 30°C for 3 hours to immobilize HEL onto the plate. After the plate was washed with a TBS buffer (pH 7.0), this was blocked with a TBS buffer containing Block Ace (manufactured by Dainippon Pharmaceutical Co., Ltd.) (at 4°C, overnight). After washed with TBS, incubation was performed at room temperature for 3 hours using TBS (containing 10% Block Ace) containing 24 µg of mouse anti-HEL scFv obtained in Example 6. After washed with TBS, incubation (2 hours, 30°C) was performed using a TBS buffer comprising an Antimouse IgG-HRP conjugated (manufactured by Funakoshi Co., Ltd) as a secondary antibody. After washed with TBS, 100 µL of an ABTS solution (manufactured by Funakoshi Co., Ltd) as a substrate for HRP was added, followed by incubation for 30 minutes and measurement of OD405. The obtained results are shown in Fig. 8. Since absorbance is increased (A) depending on a concentration of HEL immobilized on a plate, it was confirmed that the resulting scFv is bound with an antigen. On the other hand, when the same concentration of chymotrypsin inhibitor (□) was used in place of HEL, increase in absorbance was not observed. It seems that this demonstrates that the antibody obtained by expression is specifically bound with the antigen.

### Industrial applicability

Since the present invention comprises the aforementioned essential features, it enables to prevent formation of an inclusion body which has previously been a problem in a protein expression system using bacteria, yeast and insect cell, and express a native-type protein in a soluble fraction at a large amount. Whereby, labor of re-folding an inclusion body into a native-type protein in vitro as previous becomes unnecessary.

## Claims

1. An expression vector,
which comprises:
(a) a first coding region encoding peptidyl-prolyl cis-trans isomerase (PPlase) having molecular chaperone activity; and
(b) a region having at least one restriction enzyme site in which a second coding region encoding a desired protein can be inserted;
wherein the PPlase having molecular chaperone activity is archaebacterial FKBP-type PPlase, or comprises an IF domain and/or C-terminal domain of archaebacterial FKBP-type PPlase; and
wherein the first coding region is operatively linked to a promoter, and the restriction enzyme site is in the same reading frame as the first coding region, and is downstream of the first coding region.

2. The expression vector according to claim 1,
which has a region being between a first coding region and a region having at least one restriction enzyme site in which a second coding region can be inserted, and is translated in the same reading frame to be a protease digestion site.

3. An expression vector,
wherein a second coding region encoding a desired protein is inserted into the expression vector according to claim 1 or 2.

4. The expression vector according to claim 3,
wherein the archaebacterial FKBP-type PPlase is short type FKBP-type PPlase.

5. The expression vector according to claim 3 or 4,
wherein the second coding region has a nucleotide sequence encoding a monoclonal antibody.

6. The expression vector according to claim 3 or 4,
wherein the second coding region has a nucleotide sequence encoding a membrane protein.

7. A host,
which contains the expression vector according to any of claims 1 to 6.

8. The host according to claim 7,
which is Escherichia coli.

9. A fused protein,
which comprises peptidyl-prolyl cis-trans isomerase (PPlase) having molecular chaperone activity and a desired protein;
wherein the PPlase having molecular chaperone activity is archaebacterial FKBP-type PPlase, or comprises an IF domain and/or C-terminal domain of archaebacterial FKBP-type PPlase;
wherein the fused protein is obtainable from expression of an expression vector according to any of claims 1-6.

10. The fused protein according to claim 9,
which comprises a protease digestion site between PPlase having molecular chaperone activity and a desired protein.

11. A process for producing a fused protein comprising PPlase having molecular chaperone activity and a desired protein,
which comprises making the expression vector according to claim 3 or 4.

12. The process for producing a fused protein according to claim 11,
which comprises culturing the host containing the expression vector under condition of expression of the expression vector, and making express the fused protein in a cytoplasm.

13. The process for producing a fused protein according to claim 11.
which comprises providing a region being transcribed and translated to be a signal sequence at a 5' terminus of a first coding region or a 5' terminus of a second coding region of the expression vector, and culturing a host containing the expression vector under condition of expression of the expression vector to express the fused protein in a periplasm or a medium.

14. The process for producing a fused protein according to claim 11,
which comprises making the expression vector express the fused protein in a cell-free translation system.

15. The process for producing a fused protein according to any of claims 11 to 14,
wherein the fused protein is adsorbed on a carrier harboring macrolide, cyclosporin, juglone or its analogous compound inhibiting PPlase activity, and then the carrier is recovered and the fused protein is recovered from the carrier.

16. A process for producing a desired protein,
which comprises digesting the fused protein comprising a protease digestion site obtained by the process according to any of claims 11 to 14 with a protease digesting a protease digestion site.

## Patentansprüche

1. Ein Expressionsvektor,
der umfasst:
(a) eine erste Kodierungsregion, die Peptidylprolyl-cis/trans-Isomerase (PPlase) kodiert und molekulare Chaperon-Aktivität aufweist; und
(b) eine Region, die mindestens eine Restriktionsenzymstelle aufweist, in die eine zweite Kodierungsregion, die ein gewünschtes Protein kodiert, eingefügt werden kann;
wobei die PPlase, die molekulare Chaperon-Aktivität aufweist, archaebakterielle PPlase vom Typ FKBP ist oder eine IF-Domäne und/oder C-terminale Domäne der archaebakteriellen PPlase vom Typ FKBP umfasst; und
wobei die erste Kodierungsregion betrieblich mit einem Promotor verbunden ist, und wobei die Restriktionsenzymstelle sich in dem gleichen Leseraster befindet wie die erste Kodierungsregion und sich abwärts der ersten Kodierungsregion befindet.

2. Der Expressionsvektor gemäß Anspruch 1,
der eine Region aufweist, die sich zwischen einer ersten Kodierungsregion und einer Region befindet, die mindestens eine Restriktionsenzymstelle aufweist, in die eine zweite Kodierungsregion eingefügt werden kann, und dessen Translation in dem gleichen Leseraster als Proteaseverdauungsstelle erfolgt.

3. Der Expressionsvektor,
wobei eine zweite Kodierungsregion, die ein gewünschtes Protein kodiert, in den Expressionsvektor gemäß Anspruch 1 oder 2 eingefügt wird.

4. Der Expressionsvektor gemäß Anspruch 3,
wobei die archaebakterielle PPlase vom Typ FKBP eine Kurztyp-PPlase vom Typ FKBP ist.

5. Der Expressionsvektor gemäß Anspruch 3 oder 4,
wobei die zweite Kodierungsregion eine Nucleotidsequenz aufweist, die einen monoklonalen Antikörper kodiert.

6. Der Expressionsvektor gemäß Anspruch 3 oder 4,
wobei die zweite Kodierungsregion eine Nucleotidsequenz aufweist, die ein Membranprotein kodiert.

7. Ein Host,
der den Expressionsvektor gemäß einem beliebigen der Ansprüche 1 bis 6 enthält.

8. Der Host gemäß Anspruch 7,
der Escherichia coli ist.

9. Ein fusioniertes Protein,
das Peptidylprolyl-cis/trans-Isomerase (PPlase), die molekulare Chaperon-Aktivität aufweist, und ein gewünschtes Protein umfasst;
wobei die PPlase, die molekulare Chaperon-Aktivität aufweist, eine archaebakterielle PPlase vom Typ FKBP ist oder eine IF-Domäne und/oder C-terminale Domäne der archaebakteriellen PPlase vom Typ FKBP umfasst;
wobei das fusionierte Protein durch Expression eines Expressionsvektors gemäß einem beliebigen der Ansprüche 1 bis 6 gewonnen werden kann.

10. Das fusionierte Protein gemäß Anspruch 9,
das eine Proteaseverdauungsstelle zwischen PPlase, die molekulare Chaperon-Aktivität aufweist, und einem gewünschten Protein umfasst.

11. Ein Prozess zur Herstellung eines fusionierten Proteins, das PPlase, die molekulare Chaperon-Aktivität aufweist, und ein gewünschtes Protein umfasst,
der die Herstellung des Expressionsvektors gemäß Anspruch 3 oder 4 umfasst.

12. Der Prozess zur Herstellung eines fusionierten Proteins gemäß Anspruch 11,
der das Anlegen einer Kultur des Hosts, der den Expressionsvektor enthält, unter Bedingungen für die Expression des Expressionsvektors und das Bewerkstelligen der Expression des fusionierten Proteins in einem Zytoplasma umfasst.

13. Der Prozess zur Herstellung eines fusionierten Proteins gemäß Anspruch 11,
der das Bereitstellen einer Region, deren Transkription und Translation zu einer Signalsequenz an einer 5'-Endstelle einer ersten Kodierungsregion oder einer 5'-Endstelle einer zweiten Kodierungsregion des Expressionsvektors im Gange ist, und das Anlegen einer Kultur des Hosts, der der Expressionsvektor enthält, unter Bedingungen für die Expression des Expressionsvektors, zum Exprimieren des fusionierten Proteins in einem Periplasma oder Medium umfasst.

14. Der Prozess zur Herstellung eines fusionierten Proteins gemäß Anspruch 11,
der die Bewerkstelligung der Expression des fusionierten Proteins durch den Expressionsvektor in einem zellfreien Translationssystem umfasst.

15. Der Prozess zur Herstellung eines fusionierten Proteins gemäß einem beliebigen der Ansprüche 11 bis 14,
wobei das fusionierte Protein auf einem Träger adsorbiert wird, der ein Makrolid, Ciclosporin, Juglon oder deren Analogverbindung beherbergt, das die PPlase-Aktivität hemmt, und der Träger dann wieder zurückgewonnen wird und das fusionierte Protein wieder von dem Träger zurückgewonnen wird.

16. Der Prozess zur Herstellung eines gewünschten Proteins,
der das Verdauen des fusionierten Proteins umfasst, das eine Proteaseverdauungsstelle umfasst, die durch den Prozess gemäß einem beliebigen der Ansprüche 11 bis 14 gewonnen wird, wobei eine Protease eine Proteaseverdauungsstelle verdaut.

## Revendications

1. Vecteur d'expression
qui comprend :
(a) une première région codante de l'isomérase peptidyl-prolyl cis-trans (PPlase) possédant une activité de chaperon moléculaire ; et
(b) une région possédant au moins un site d'enzymes de restriction permettant l'insertion d'une deuxième région codante d'une protéine désirée ;
dans laquelle la PPlase possédant une activité de chaperon moléculaire est une PPlase de type protéine de liaison des génomes archaebactériens FK ou comprend un domaine IF et/ou un domaine C-terminal de la PPlase de type protéine de liaison du génome archaebactérien FK ; et
dans lequel la première région codante est fonctionnellement liée à un promoteur, et le site d'enzymes de restriction se trouve dans le même cadre de lecture que la première région codante et est situé en aval de la première région codante.

2. Vecteur d'expression selon la revendication 1,
qui possède une région située entre une première région codante et une région possédant au moins un site d'enzymes de restriction permettant l'insertion d'une deuxième région codante et est traduite dans le même cadre de lecture comme étant un site de digestion par la protéase.

3. Vecteur d'expression,
dans lequel une deuxième région codante d'une protéine désirée est insérée dans le vecteur d'expression selon la revendication 1 ou 2.

4. Vecteur d'expression selon la revendication 3,
dans lequel la PPlase de type protéine de liaison des génomes archaebactériens FK est une PPlase de type protéine de liaison des génomes archaebactériens FK de type à chaîne courte.

5. Vecteur d'expression selon la revendication 3 ou 4,
dans lequel la deuxième région codante possède une séquence nucléotidique codante d'un anticorps monoclonal.

6. Vecteur d'expression selon la revendication 3 ou 4,
dans lequel la deuxième région codante possède une séquence nucléotidique codante d'une protéine de membrane.

7. Hôte,
qui contient le vecteur d'expression selon l'une quelconque des revendications 1 à 6.

8. Hôte selon la revendication 7,
qui est Escherichia coli.

9. Protéine fusionnée,
qui comprend une isomérase peptidyl-propyl cis-trans (PPlase) possédant une activité de chaperon moléculaire et une protéine désirée ;
dans laquelle la PPlase possédant une activité de chaperon moléculaire est une PPlase de type protéine de liaison des génomes archaébactériens FK, ou comprend un domaine IF et/ou un domaine C-terminal de la PPlase de type protéine de liaison des génomes archaebactériens FK;
dans laquelle la protéine fusionnée est obtensible à partir d'une expression d'un vecteur d'expression selon l'une quelconque des revendications 1 à 6.

10. Protéine fusionnée selon la revendication 9,
qui comprend un site de digestion par la protéase entre la PPlase possédant une activité de chaperon moléculaire et une protéine désirée.

11. Procédé de production d'une protéine fusionnée comprenant une activité de chaperon moléculaire et une protéine désirée,
qui comprend le vecteur d'expression selon la revendication 3 ou 4.

12. Procédé de production d'une protéine fusionnée selon la revendication 11,
qui comprend la culture de l'hôte contenant le vecteur d'expression sous une condition d'expression du vecteur d'expression, et provoquant l'expression de la protéine fusionnée dans un cytoplasme.

13. Procédé de production d'une protéine fusionnée selon la revendication 11,
qui comprend la fourniture d'une région qui est transcrite et traduite comme étant une séquence de signaux à un 5'-terminal d'une première région codante ou à un 5'-terminal d'une deuxième région codante d'un vecteur d'expression et la culture d'un hôte contenant le vecteur d'expression sous une condition d'expression du vecteur d'expression pour exprimer la protéine fusionnée dans un périplasme ou un milieu de culture.

14. Procédé de production d'une protéine fusionnée selon la revendication 11,
qui comprend l'expression par le vecteur d'expression de la protéine fusionnée dans un système de traduction exempt de cellules.

15. Procédé de production d'une protéine fusionnée selon l'une quelconque des revendications 11 à 14,
dans lequel la protéine fusionnée est adsorbée sur un macrolide hébergeant un porteur, une cyclosporine, une juglone ou son composé analogue inhibant une activité PPlase, et le porteur est ensuite récupéré et la protéine fusionnée est récupérée du porteur.

16. Procédé de production d'une protéine désirée,
qui comprend la digestion de la protéine fusionnée comprenant un site de digestion par la protéase obtenu par le procédé selon l'une quelconque des revendications 11 à 14 avec une protéase digérant un site de digestion par la protéase.
